(19) Europäisches Patentamt
European Patent Office
Office européen des brevets

(11) EP 2 668 174 B1

(12) EUROPÄISCHE PATENTSCHRIFT

(45) Veröffentlichungstag und Bekanntmachung des Hinweises auf die Patenterteilung:
**31.05.2017 Patentblatt 2017/22**

(21) Anmeldenummer: **11808173.6**

(22) Anmeldetag: **30.12.2011**

(51) Int Cl.:
***C07D 307/06*** *(2006.01)* ***C07D 319/04*** *(2006.01)*

(86) Internationale Anmeldenummer:
**PCT/EP2011/006608**

(87) Internationale Veröffentlichungsnummer:
**WO 2012/100809 (02.08.2012 Gazette 2012/31)**

# (54) FLÜSSIGKRISTALLINE VERBINDUNGEN UND FLÜSSIGKRISTALLINE MEDIEN

LIQUID-CRYSTALLINE COMPOUNDS AND LIQUID-CRYSTALLINE MEDIA

COMPOSÉS CRISTAL LIQUIDE ET MILIEUX CRISTAL LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.01.2011 DE 102011009338**

(43) Veröffentlichungstag der Anmeldung:
**04.12.2013 Patentblatt 2013/49**

(73) Patentinhaber: **Merck Patent GmbH**
**64293 Darmstadt (DE)**

(72) Erfinder:
- **JANSEN, Axel**
  **64293 Darmstadt (DE)**
- **BREMER, Matthias**
  **64295 Darmstadt (DE)**
- **WITTEK, Michael**
  **64390 Erzhausen (DE)**
- **HAENSEL, Helmut**
  **64367 Muehltal (DE)**
- **RILLICH, Malgorzata**
  **64291 Darmstadt (DE)**
- **SPRANG, Julia**
  **61276 Weilrod (DE)**

(56) Entgegenhaltungen:

| | |
|---|---|
| **EP-A1- 0 819 685** | **EP-A2- 0 782 995** |
| **WO-A1-2006/125527** | **WO-A1-2010/134430** |
| **DE-A1- 10 318 420** | **US-A1- 2012 099 039** |
| **US-B2- 7 109 381** | |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 668 174 B1

## Beschreibung

Die vorliegende Erfindung betrifft flüssigkristalline Verbindungen mit einem O-heterocyclischen Ring, drei teilweise fluorierten Benzolringen und einer $-CF_2O-$ Brücke zwischen den Ringen. Die Erfindung betrifft außerdem damit hergestellte flüssigkristalline Medien und diese Medien enthaltende Flüssigkristall-Anzeigevorrichtungen (LC-Displays).

Flüssigkristalline Medien werden seit längerem in LC-Displays genutzt, um Informationen anzuzeigen. Verbindungen mit 4 oder 5 Ringen, darunter einem O-heterocyclischen Ring und einer $-CF_2O-$ Brücke werden schon für flüssigkristalline Anzeigevorrichtungen vorgeschlagen, z.B. in den Druckschriften EP 0 819 685 A1, JP 10-251186 A, EP 2 028 252 A1, WO 2004/048501 A1, US 2009/0237610 A1, WO 2010/134430 A1 und US 2009/0059157 A1. In der Druckschrift US 2009/0059157 A1 werden außerdem LC-Displays, die in der optisch isotropen blauen Phase arbeiten, offenbart, sowie eine Vielzahl möglicher Verbindungen als flüssigkristalline Komponente. Die erfindungsgemäßen Verbindungen im Speziellen lassen sich diesen Offenbarungen nicht entnehmen.

Neben den dem Fachmann gut bekannten Displays mit nematischen Flüssigkristallen werden auch zunehmend Anwendungen entwickelt, die auf Medien mit einer blaue Phase basieren. Diese zeichnen sich durch besonders niedrige Schaltzeiten aus. Bei Displayanwendungen, bei denen elektrooptische Effekte der flüssigkristallinen blauen Phasen genutzt werden, sind insbesondere die Parameter $\Delta\varepsilon$ und $\Delta n$ von entscheidender Bedeutung.

Grundlage für schnelle Schaltvorgänge in diesen Phasen ist der sog. Kerr-Effekt. Der Kerr-Effekt ist die Änderung der Doppelbrechung eines optisch transparenten und isotropen Materials hervorgerufen durch ein äußeres elektrisches Feld. Die Änderung der Doppelbrechung wird durch folgende Gleichung wiedergegeben.

$$\Delta n_{induziert} = \lambda \cdot K \cdot E^2$$

Dabei ist $\Delta n_{induziert}$ die induzierte Doppelbrechung, K ist die Kerr-Konstante und E das angelegte elektrische Feld. $\lambda$ steht für die Wellenlänge. Außergewöhnlich hohe Kerr-Konstanten werden für Materialien in der blauen Phase beobachtet.

*Kikuchi* et al. beschreiben die Abhängigkeit der Kerr-Konstante von den LC-Materialeigenschaften [H. Kikuchi et al., Appl. Phys. Lett. 2008, 92, 043119.]. Danach ist die Kerr-Konstante proportional zum Produkt aus Doppelbrechung und dielektrischer Anisotropie des flüssigkristallinen Mediums.

$$K \sim \Delta n \cdot \Delta\varepsilon$$

Für schnelle Schaltprozesse und geringe Schaltspannungen werden Materialien mit hohen Werten für die Kerr-Konstante und somit hohen Werten für das Produkt $\Delta n \cdot \Delta\varepsilon$ benötigt.

Eine Aufgabe der vorliegenden Erfindung ist es, Verbindungen mit vorteilhaften Eigenschaften für den Einsatz in flüssigkristallinen Medien zur Verfügung zu stellen. Insbesondere sollen Sie für den Einsatz in Displays geeignet sein, die Medien mit polymerstabilisierten blauen Phasen verwenden. Hier werden Materialien benötigt, die ein schnelles Schalten ermöglichen, eine gute 'Voltage-holding-ratio' (VHR) aufweisen, geringe Spannungen für den Schaltprozess ($V_{op}$) benötigen, hohe Klärpunkte besitzen, eine geringe Hysterese zeigen, einen geringen Memory-Effekt aufweisen und stabil gegenüber Belastungen durch Licht- und Temperatur sind. Darüber hinaus sollten die Einzelverbindungen über eine ausreichende Löslichkeit in nematischen FK-Medien verfügen, bzw. selbst einen breiten nematischen Phasenbereich aufweisen.

Eine weitere Aufgabe der Erfindung ist es, flüssigkristalline Medien bereitzustellen, die im Wesentlichen frei von Esterverbindungen oder Nitrilen sind, um den elektrischen Widerstand der Mischungen und dessen Langzeitstabilität zu erhöhen. Die bisher bekannten flüssigkristallinen Medien für den Betrieb in der optisch isotropen blauen Phase enthalten bisweilen z.B. Verbindungen der Formel,

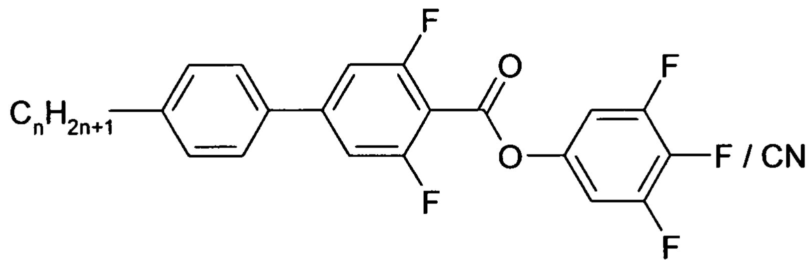

worin n = 3 - 5 ist,
die ganz oder teilweise durch Verbindungen mit ähnlichen physikalischen Eigenschaften und ausgeprägter Stabilität ersetzt werden sollen.

Diese Aufgaben werden erfindungsgemäß gelöst durch Verbindungen der allgemeinen Formel **I**. Überraschenderweise wird auch gefunden, dass mit den erfindungsgemäßen Verbindungen flüssigkristalline Medien mit einem geeigneten, nematischen Phasenbereich, hoher dielektrischer Anisotropie $\Delta\varepsilon$ und hohem $\Delta n$ verwirklicht werden können, welche die Nachteile der Materialien des Standes der Technik nicht oder zumindest nur in erheblich geringerem Maße aufweisen. In weiten Teilen gleiche Anforderungen werden an hochpolare Substanzen für rein nematische Displays gestellt.

Die Erfindung umfasst Verbindungen der Formel I,

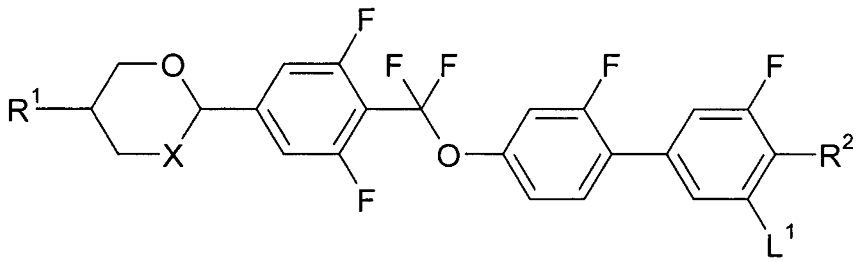

I

worin

$L^1$ F

X O

$R^1$ einen unsubstituierten Alkylrest mit 1 bis 15 C-Atomen, wobei in diesem Rest auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)- oder -O- so ersetzt sein können, dass O-Atome nicht direkt miteinander verknüpft sind,

und

$R^2$ $CF_3$ oder $OCF_3$

bedeuten.

Die erfindungsgemäßen Verbindungen besitzen einen relativ hohen Klärpunkt, eine außergewöhnlich hohe dielektrische Anisotropie ($\Delta\varepsilon$), eine hohe optische Anisotropie ($\Delta n$), sowie eine geringe Rotationsviskosität. Sie besitzen, allein oder in Mischung mit weiteren mesogenen Komponenten, über einen breiten Temperaturbereich eine nematische Phase. Diese Eigenschaften machen sie geeignet für die Verwendung in flüssigkristallinen Medien, z.B. für Anzeigen vom Typ TN-TFT, IPS, FFS, 'blue-phase', HT-VA, etc., gekennzeichnet durch Medien mit positiver dielektrischer Anisotropie, die dem Fachmann vertraut sind. Besonders geeignet sind sie für die Anwendung in Medien im Bereich der blauen Phase.

Der Rest $R^1$ bedeutet bevorzugt ein unsubstituiertes Alkyl mit 1 bis 9 C-Atomen, und ganz besonders bevorzugt ein geradkettiges Alkyl mit bis zu 9 C-Atomen.

Besonders bevorzugt bedeutet der Rest $R^2$ $CF_3$. Diese Verbindungen besitzen einen besonders hohen Wert für das Produkt $\Delta n \cdot \Delta\varepsilon$.

Beispielhafte bevorzugte Ausführungsformen der Erfindung sind daher die folgenden Strukturen:

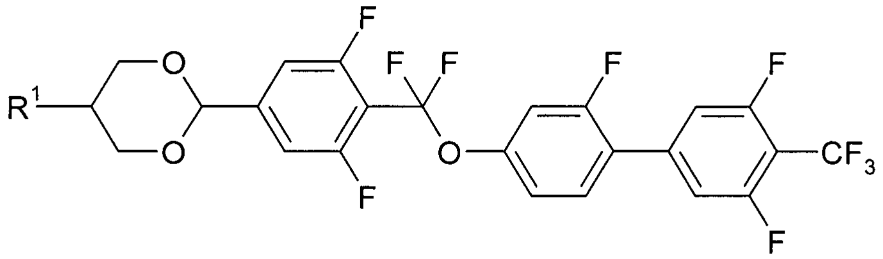

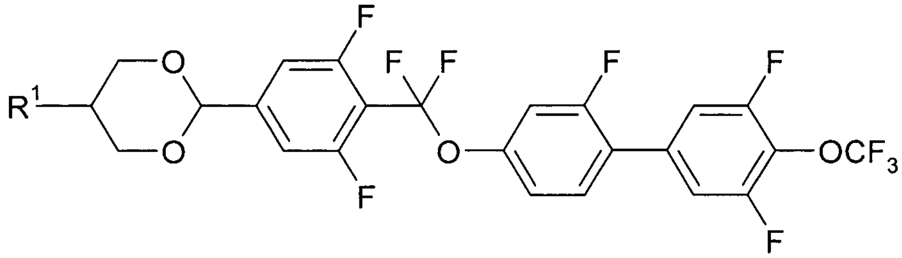

worin $R^1$ wie oben definiert ist, bevorzugt eine geradkettige Alkylgruppe der Formel $-C_nH_{2n+1}$ worin n = 1, 2, 3, 4, 5, 6 oder 7, insbesondere 3, ist.

Die Verbindungen der Formel I können vorteilhaft wie an der folgenden beispielhaften Synthese ersichtlich hergestellt werden (Schemata 2-3).

Die Verbindungen **I** werden vorteilhafterweise ausgehend von den Aldehyden **1** hergestellt (Schema 2).

Ist X = $CH_2$, handelt es sich um Vergleichsverbindungen und um Tetrahydropyrane, so erfolgt die Synthese durch Umsetzung der Aldehyde **1** mit 3-Propenylalkohlen **2.** In einer Cyclisierung nach Art der Prins-Reaktion werden die Verbindungen **3** gebildet. Durch reduktive Eliminierung, hier mittels Hydrierung an Palladium-Kohle in Gegenwart von Triethylamin, werden die Tetrahydropyrane **4** (= Verbindungen **I** mit X = $-CH_2-$) erhalten.

**Schema 1:** Synthese der Vergleichsverbindungen in denen X = $-CH_2-$ ist (= **4** im Speziellen).

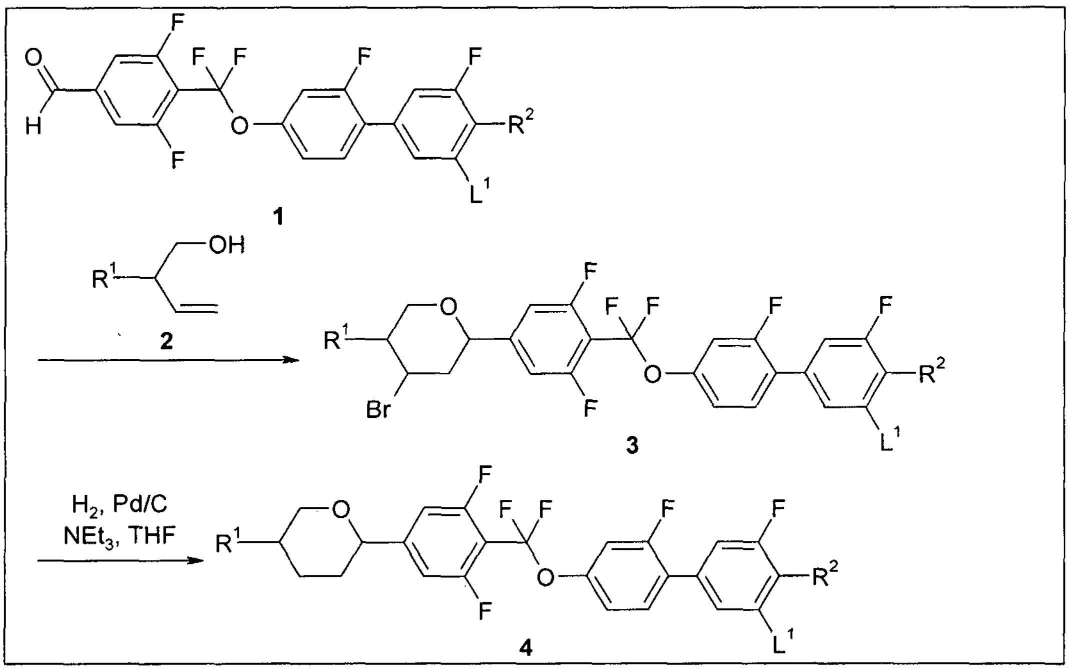

Die Reaktion des Aldehyds mit dem Homoallylalkohol erfolgt mit Hilfe einer halogenhaltigen Säure, vorzugsweise einer halogenhaltigen Lewissäure, in einem organischen Lösungsmittel wie z. B. Dichlormethan. Eine ähnliche Reaktion eines Aldehyds mit einem Alkenol wird bei J.O. Metzger et al. und den darin zitierten Stellen beschrieben (Bull. Soc. Chem. Belg. (1994), 103, 393-7).

Das Verfahren lässt sich vorteilhaft in Gegenwart einer Lewis-Säure der Formeln $M(X^1)_n$ oder $R^5M(X^1)_{n-1}$ durchführen, wobei

M B, Al, In, Sn, Ti, Fe, Zn, Zr, Au oder Bi bedeutet;
$X^1$ Cl, Br oder I bedeutet;
$R^5$ einen geradkettigen oder verzweigten Alkylrest mit 1 bis 10 Kohlenstoffatomen bedeutet; und
n eine ganze Zahl 2, 3 oder 4 ist und so ausgewählt ist, dass sie gleich der formalen Oxidationszahl von M ist.

Beispiele für besonders geeignete Lewissäuren sind Halogenide der Elemente Bor, Aluminium, Eisen, Zink oder Bismut. Ganz besonders geeignet sind z. B. $AlCl_3$ oder $BiBr_3$. Alternativ können an Stelle der Lewis-Säure auch Brönsted-Säuren wie Bromwasserstoff (HBr) eingesetzt werden.

Die reduktive Eliminierung des Halogens an den entstehenden 4-Halogentetrahydropyranen kann in verschiedenen Varianten erfolgen (vgl. WO 2006/125526).

Dioxane **I** (X = O) werden durch Umsetzung der Aldehyde **1** mit 1,3-Propandiolen **5** erhalten. Dies erfolgt typischerweise säurekatalysiert.

**Schema 2:** Synthese der Verbindungen **I** (= **6** im Speziellen).

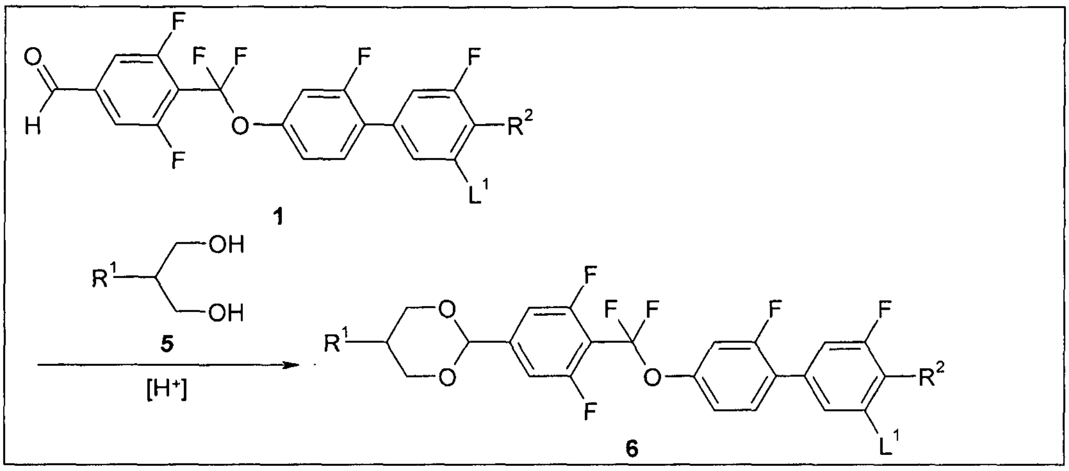


Als Säure wird bevorzugt eine Sulfonsäure, besonders bevorzugt p-Toluolsulfonsäure oder Trifluormethansulfonsäure, zugesetzt. In einer bevorzugten Verfahrensweise wird unter den angegebenen Reaktionsbedingungen das entstehende Wasser durch azeotrope Destillation aus dem Reaktionsgemisch entfernt. Bevorzugte Verfahren zur Bildung von Dioxanen sind ebenso durch Lewis-Säuren katalysierte Varianten der Acetalbildung. Besonders bevorzugt sind auch besonders milde Verfahren unter Zuhilfenahme katalytischer Mengen von Ruthenium- oder Indiumhalogeniden, insbesondere von $RuCl_3$ und $InCl_3$ (vgl. Literatur: B.C. Janu et al., Adv. Synth. Catal. (2004), 346, 446-50; J.-Y. Qi et al., Tetr. Lett. (2004), 45, 7719-21; S.K. De, R.A. Gibbs, Tetr. Lett. (2004), 45, 8141-4). Die milden Reaktionsbedingungen eignen sich besonders für Verbindungen mit Strukturteilen vom Typ der Ether und für säureempfindliche Gruppen.

Die benötigten Ausgangsmaterialien können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Die Ausgangsmaterialien **1** werden vorteilhafterweise wie in **Schema 3** dargestellt synthetisiert.

## Schema 3: Synthese der Verbindungen 1

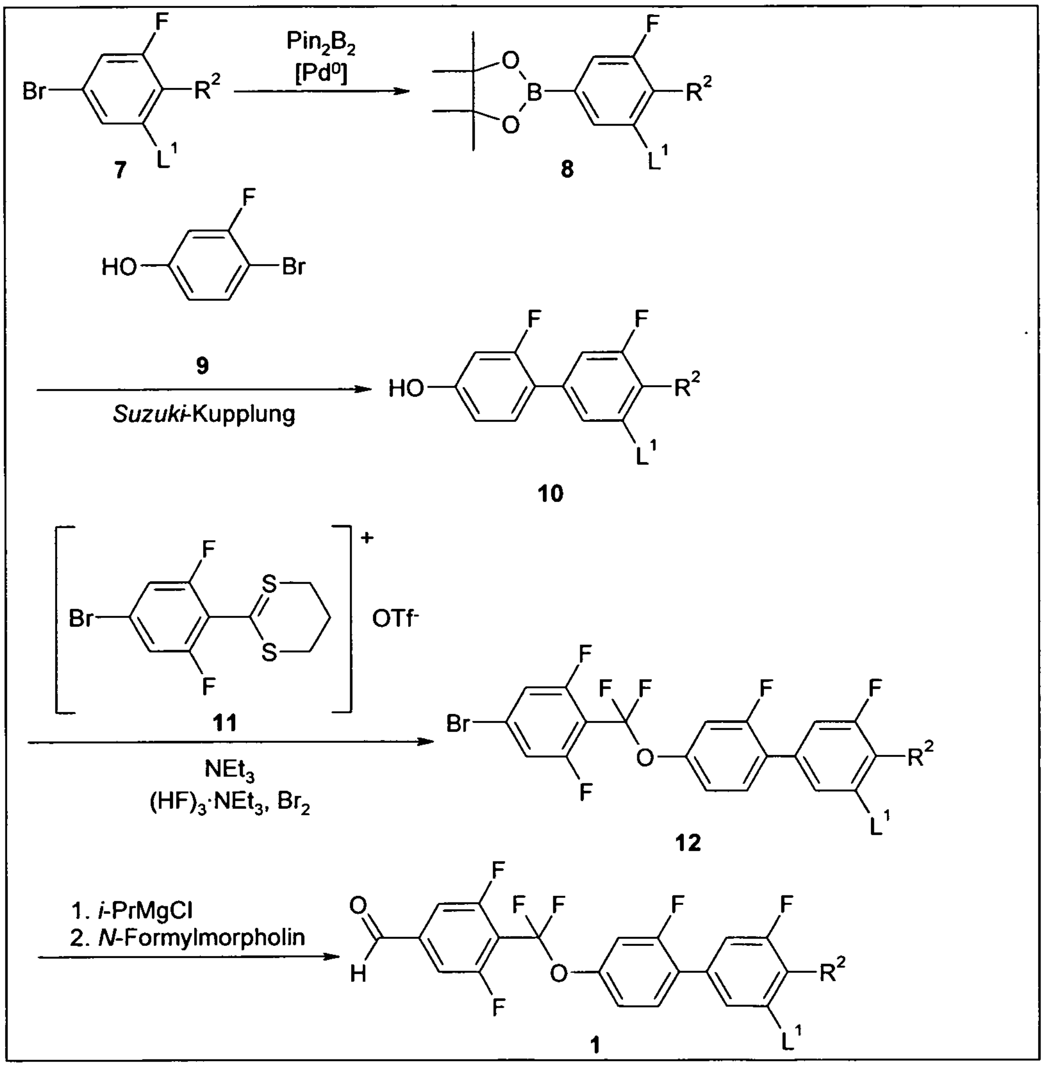


Aus den entsprechenden Bromiden **7** werden zunächst die Boronsäureester **8** hergestellt. Dies erfolgt durch eine Palladiumvermittelte Borylierung mit Bis(pinakolat)dibor ($Pin_2B_2$). Die Verbindungen werden dann mit den *para*-Bromphenolen **9** gekuppelt (*Suzuki*-Kupplung). Die Phenole **10** werden in Gegenwart von Base mit dem Dithianyliumsalz **11** umgesetzt, und das jeweilige Addukt wird direkt einer oxidativen Desulfurierung unterzogen [P. Kirsch, M. Bremer, A. Taugerbeck, T.

Wallmichrath, Angew. Chem. Int. Ed. 2001, 40, 1480-1484]. Auf diese Weise werden die Verbindungen **12** erhalten. Halogen-Metall-Austausch, bevorzugt mit Isopropylmagnesiumchlorid gefolgt von der Umsetzung mit einem Formylierungsreagenz (z.B. Formylmorpholin oder DMF) ergibt die Verbindungen **1.**

Die Erfindung umfasst daher auch ein Verfahren zur Herstellung von Verbindungen der Formel I, das einen Reaktionsschritt umfasst, worin ein Aldehyd der Formel A:

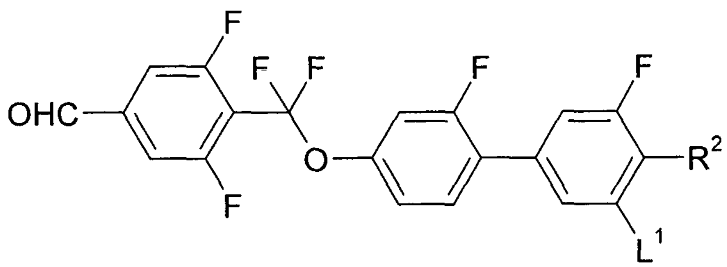

A

worin $R^2$ und $L^1$ wie für Formel I definiert sind, mit einer Verbindung der Formel B

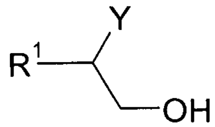

B

worin

$R^1$ wie für Formel I definiert ist und
Y einen Rest der Formel $-CH_2-OH$

bedeutet, unter geeigneten Reaktionsbedingungen umgesetzt wird.

In einer bevorzugten Ausführungsform umfasst das Verfahren zur Herstellung der Verbindungen der Formel I daher einen ersten Reaktionsschritt, der dadurch gekennzeichnet ist, dass die Verbindungen A und B in Gegenwart einer Säure umgesetzt werden.

Die flüssigkristallinen Medien gemäß der vorliegenden Erfindung enthalten eine oder mehrere Verbindungen der Formel I und optional mindestens eine weitere, vorzugsweise mesogene Verbindung. Das Flüssigkristallmedium enthält daher bevorzugt zwei oder mehr Verbindungen. Bevorzugte Medien umfassen die bevorzugten Verbindungen der Formel I.

Die erfindungsgemäßen flüssigkristallinen Medien besitzen vorzugsweise eine positive dielektrische Anisotropie. Sie lassen sich so konzipieren, dass sie eine sehr hohe dielektrische Anisotropie kombiniert mit hohen optischen Anisotropien besitzen.

Bevorzugte weitere Verbindungen für die flüssigkristallinen Medien gemäß der Erfindung sind ausgewählt aus den Verbindungen der Formel II und III:

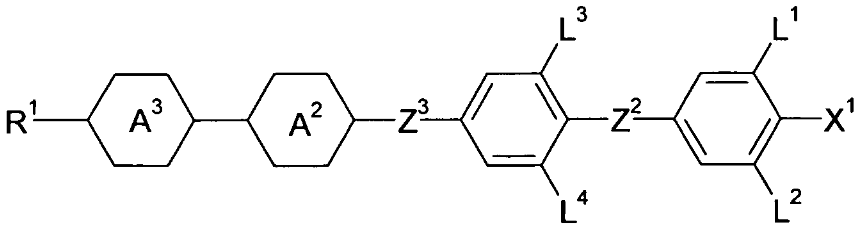

II

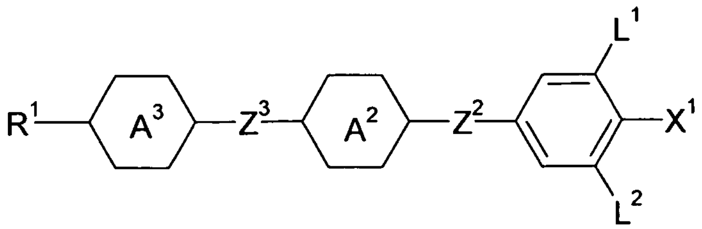

III

worin

$R^1$ jeweils unabhängig voneinander einen unsubstituierten Alkylrest mit 1 bis 15 C-Atomen, wobei in diesem Rest auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)- oder -O- so ersetzt sein können, dass O-Atome nicht direkt miteinander verknüpft sind, bevorzugt einen geradkettigen Alkylrest mit 2 bis 7 C-Atomen,

$A^2$, $A^3$ unabhängig voneinander

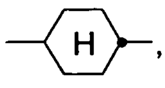, 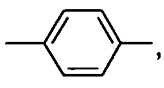, 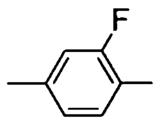, 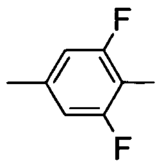,

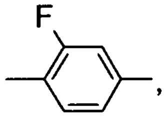, 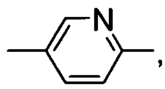, 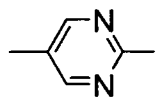, 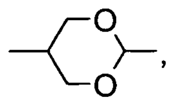,

$Z^2$, $Z^3$ unabhängig voneinander eine Einfachbindung, $CF_2O$, $CH_2CH_2$, $CF_2CH_2$, $CF_2CF_2$, CFHCFH, $CFHCH_2$, (CO)O, $CH_2O$, C≡C, CH=CH, CF=CH, CF=CF; wobei unsymmetrische Bindeglieder (z.B. $CF_2O$) in beide möglichen Richtungen orientiert sein können,

$X^1$ F, Cl, CN, oder Alkyl, Alkenyl, Alkenyloxy, Alkylalkoxy oder Alkoxy mit 1 bis 3 C-Atomen, welches durch F ein- oder mehrfach substituiert ist, und

$L^1$ bis $L^4$ H oder F,

bedeuten.

Bevorzugt enthalten die flüssigkristallinen Medien zwischen 10 und 50 Gew.-% an Verbindungen der Formel I. Bei mehr als 10 % Gesamtgehalt werden bevorzugt zwei oder mehr Verbindungen der Formel I mit unterschiedlichen Kettenlängen im Rest $R^1$ eingesetzt.

Bevorzugt enthalten die flüssigkristallinen Medien zwischen 20 und 40 Gew.-% an Verbindungen der Formel II. Die Verbindungen der Formel III werden bevorzugt, sofern vorhanden, mit bis zu 20 Gew.-% eingesetzt. Die restlichen sonstigen Verbindungen, sofern vorhanden, sind ausgewählt aus weiteren Verbindungen mit hoher dielektrischer Anisotropie, hoher optischer Anisotropie und vorzugsweise mit hohem Klärpunkt.

Flüssigkristalline Medien mit überproportional hohen dielektrischen Anisotropien lassen sich durch einen hohen Anteil an den Verbindungen der Formel I erreichen, vorzugsweise ergänzt durch Verbindungen der Formeln II und III.

Bevorzugte Verbindungen der Formel II sind solche der Formel IIa:

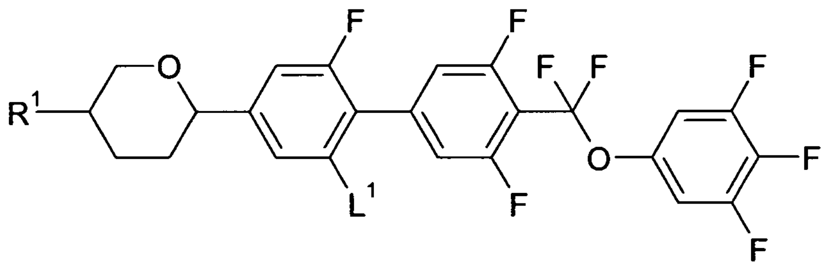

IIa

worin $R^1$ und $L^1$ wie für Formel II definiert sind.

Bevorzugte Verbindungen der Formel III sind solche der Formel IIIa oder IIIb:

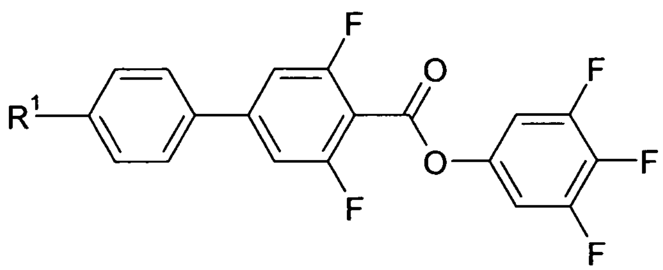

IIIa

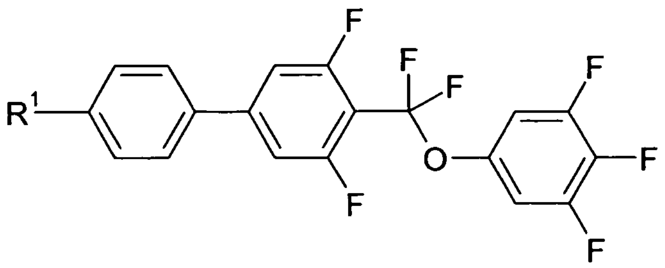

IIIb

worin $R^1$ wie für Formel III definiert ist.

Ein weiterer Gegenstand der Erfindung ist die Verwendung der Verbindungen der Formel I in flüssigkristallinen Medien oder in elektrooptischen Anzeigen, bevorzugt in Medien und Anzeigen mit einer optisch isotropen flüssigkristallinen Phase, vorzugsweise mit einer blauen Phase. Diese Phase ist vorzugsweise durch Polymer stabilisiert, welches vorzugsweise im flüssigkristallinen Medium aus entsprechenden Monomeren durch Polymerisieren gebildet wird. In der Regel wird der Monomeranteil des Mediums bei einer Temperatur polymerisiert, bei der es in der blauen Phase vorliegt. Dadurch verbreitert sich der Stabilitätsbereich dieser Phase. Mit den erfindungsgemäßen Verbindungen und Medien ist eine erhebliche Verbesserung der bislang erreichbaren Eigenschaften der polymerstabilisierten Medien in der blauen Phase verbunden.

Die flüssigkristallinen Medien können darüber hinaus weitere Zusätze wie Stabilisatoren, chirale Dotierstoffe und Nanopartikel enthalten. Die einzelnen, zugesetzten Verbindungen werden in Konzentrationen von vorzugsweise von 0,1 bis 6 % eingesetzt. Die Konzentrationen der einzelnen verwendeten Verbindungen liegen vorzugsweise jeweils im Bereich von 0,1 % bis 3 %. Dabei werden jedoch die Konzentrationsangaben der übrigen Bestandteile der Flüssigkristallmischungen, also der flüssigkristallinen oder mesogenen Verbindungen und gegebenenfalls der Polymerisationskomponenten, ohne Berücksichtigung der Konzentration dieser Zusatzstoffe angegeben.

Vorzugsweise enthalten die flüssigkristallinen Medien 0,01 bis 10 Gew.% eines optisch aktiven, chiralen Dotierstoffs. Dieser unterstützt die Bildung einer flüssigkristallinen blauen Phase. Für blaue Phasen werden bevorzugt chirale Dopants mit hoher HTP ('helical twisting power') eingesetzt, typischerweise im Bereich von 2-5 Gew.-%.

Die erfindungsgemäßen Medien enthalten vorzugsweise eine oder mehrere polymerisierbare Verbindungen (Monomere), oder sie sind durch ein daraus erhaltenes Polymer stabilisiert, wobei die Polymerisation vorzugsweise in der blauen Phase erfolgt.

Vorzugsweise enthalten die flüssigkristallinen Medien 0 bis 10 Gew.%, insbesondere 0,01 bis 5 Gew.% und besonders bevorzugt 0,1 bis 3 Gew.% an Stabilisatoren. Vorzugsweise enthalten die Medien einen oder mehrere Stabilisatoren ausgewählt aus 2,6-Di-tert-butylphenolen, 2,2,6,6-Tetramethylpiperidinen oder 2-Benzotriazol-2-yl-phenolen. Diese Hilfsstoffe sind dem Fachmann bekannt und kommerziell erhältlich, z. B. als Lichtschutzmittel.

Eine Ausführungsform der Erfindung ist daher auch ein Verfahren zur Herstellung eines Flüssigkristallmediums, das dadurch gekennzeichnet ist, dass eine oder mehrere Verbindungen der Formel I mit einer oder mehreren flüssigkristallinen Verbindungen, vorzugsweise ausgewählt aus den Formeln II und III, optional mit einer oder mehreren weiteren Verbindungen und optional mit einem oder mehreren Additiven gemischt wird. Optional wird der polymerisierbare Anteil des flüssigkristallinen Mediums anschließend polymerisiert.

Ein weiterer Gegenstand der vorliegenden Erfindung ist die Verwendung der erfindungsgemäßen Verbindungen oder Medien in einer elektrooptischen Vorrichtung, vorzugsweise einer Flüssigkristallanzeige, und solche Vorrichtungen selbst. Die Anzeigen arbeiten vorzugsweise wenigstens teilweise im Bereich der blauen Phase, die vorzugsweise eine polymerstabilisierte blaue Phase ist. Die Medien und Anzeigen arbeiten alternativ auch bevorzugt in der nematischen Phase.

Eine erfindungsgemäße polymerstabilisierte Vorrichtung wird vorzugsweise so hergestellt, dass man die Polymerisation der polymerisierbaren Bestandteile des Medium in der Vorrichtung selbst, also in der optoelektronischen Zelle, durchführt.

Der Aufbau der erfindungsgemäßen elektrooptischen Anzeigevorrichtung besteht vorzugsweise aus einer Zelle umfassend zwei gegenüberliegende Substrate, die das flüssigkristalline Medium umschließen, und aus in der Zelle angebrachten Elektroden. Die Elektroden sind vorzugsweise so gestaltet, dass sie ein elektrisches Feld erzeugen können, das im flüssigkristallinen Medium einen parallel zu den Substraten (bzw. senkrecht zur Lichtachse) ausgerichteten Anteil besitzt. Die Elektroden sind vorzugsweise als Kammelektroden (Interdigitalelektroden) auf einem der Substrate aufgebracht. Vorzugsweise sind eines oder beide Substrate transparent. Im Fall der Anzeigen, die in der blauen Phase arbeiten, wird durch das Anlegen einer Spannung das optisch isotrope Medium doppelbrechend. Zusammen mit entsprechend angeordneten Polarisatoren wird ein optischer Schaltvorgang erreicht.

In der vorliegenden Anmeldung beschreibt der Ausdruck dielektrisch positiv Verbindungen oder Komponenten mit $\Delta\varepsilon > 3{,}0$, dielektrisch neutral mit $-1{,}5 \leq \Delta\varepsilon \leq 3{,}0$ und dielektrisch negativ mit $\Delta\varepsilon < -1{,}5$. Die dielektrische Anisotropie

der jeweiligen Verbindung wird aus den Ergebnissen einer Lösung von 10 % der jeweiligen einzelnen Verbindung in einer nematischen Host-Mischung bestimmt. Wenn die Löslichkeit der jeweiligen Verbindung in der Host-Mischung weniger als 10 % beträgt, wird die Konzentration auf 5 % reduziert. Die Kapazitäten der Testmischungen werden sowohl in einer Zelle mit homeotroper als auch mit homogener Orientierung bestimmt. Die Schichtdicke beträgt bei beiden Zelltypen ca. 20 $\mu$m. Die angelegte Spannung ist eine Rechteckwelle mit einer Frequenz von 1 kHz und einem Effektivwert von typischerweise 0,5 V bis 1,0 V, wird jedoch stets so ausgewählt, dass sie unterhalb der kapazitiven Schwelle für die jeweilige Testmischung liegt.

Als Host-Mischung wird für dielektrisch positive Verbindungen die Mischung ZLI-4792 und für dielektrisch neutrale sowie für dielektrisch negative Verbindungen die Mischung ZLI-3086 verwendet, beide von Merck KGaA, Deutschland. Die absoluten Werte der dielektrischen Konstanten der Verbindungen werden aus der Änderung der jeweiligen Werte der Host-Mischung bei Zugabe der interessierenden Verbindungen bestimmt. Die Werte werden auf eine Konzentration der interessierenden Verbindungen von 100 % extrapoliert.

Komponenten und flüssigkristalline Medien, die bei der Messtemperatur von 20°C eine nematische Phase aufweisen, werden als solche gemessen, alle anderen werden wie Verbindungen behandelt.

Die vorliegend angegebenen Parameterbereiche schließen sämtlich die Grenzwerte ein, wenn nicht ausdrücklich etwas anderes angegeben ist.

Vorliegend gelten, wenn nicht ausdrücklich anders angegeben, die folgenden Bedingungen und Definitionen. Alle Konzentrationen sind in Massenprozent angegeben und beziehen sich jeweils auf die Gesamtmischung. Alle Temperaturen, wie z.B. derSchmelzpunkt T(K,N) bzw. T(K,S), der Übergang von der smektischen (S) zur nematischen (N) Phase T(S,N) und der Klärpunkt T (N,I) der Flüssigkristalle sind in Grad Celsius angegeben. Alle Temperaturdifferenzen sind in Differenzgraden angegeben. Alle für Flüssigkristalle typischenphysikalischen Eigenschaften werden, soweit nicht anders angegeben, nach "MerckLiquid Crystals, Physical Properties of Liquid Crystals", Stand Nov. 1997, Merck KGaA, Deutschland, bestimmt und sind für eine Temperatur von 20°C aufgeführt. Die optische Anisotropie ($\Delta$n) wird bei einer Wellenlänge von 589,3 nm bestimmt. $\Delta\varepsilon$ ist als ($\varepsilon_{\|}$ - $\varepsilon_{\perp}$) definiert, während $\varepsilon_{Drchschn.}$ ($\varepsilon_{\|}$ + 2 $\varepsilon_{\perp}$) / 3 ist.

Die Schwellenspannungen sowie alle anderen elektrooptischen Eigenschaften werden mit bei Merck KGaA, Deutschland, hergestellten Testzellen bestimmt. Die Testzellen für die Bestimmung von $\Delta\varepsilon$ besitzen eine Schichtdicke von circa 20 $\mu$m. Bei der Elektrode handelt es sich um eine kreisförmige ITO-Elektrode mit einer Fläche von 1,13 $cm^2$ und einem Schutzring. Die Ausrichtungsschichten sind SE-1211 von Nissan Chemicals, Japan, für homeotrope Ausrichtung ($\varepsilon_{\|}$) und Polyimid AL-1054 von Japan Synthetic Rubber, Japan, für homogene Ausrichtung ($\varepsilon_{\perp}$). Die Bestimmung der Kapazitäten erfolgt mit einem Frequenzgang-Analysegerät Solatron 1260 unter Verwendung einer Sinuswelle mit einer Spannung von 0,3 $V_{rms}$. Als Licht wird bei den elektrooptischen Messungen weißes Licht verwendet. Dabei wird ein Aufbau mit einem im Handel erhältlichen Gerät DMS der Fa. Autronic-Melchers, Germany verwendet. Die charakteristischen Spannungen werden unter senkrechter Beobachtung bestimmt. Die Schwellenspannung ($V_{10}$), "Mittgrau-Spannung" ($V_{50}$) und Sättigungsspannung ($V_{90}$) werden für 10 %, 50 % bzw. 90 % relativen Kontrast bestimmt.

Die Werte für die Komponenten der Eigenschaften senkrecht bzw. and parallel zum Direktor des Flüssigkristalls werden durch Orientierung des Flüssigkristalls in einem Magnetfeld erhalten. Dazu wird das Magnetfeld eines Permanentmagneten verwendet. Die Stärke des Magnetfelds beträgt 0,35 Tesla. Die Orientierung des Magneten wird entsprechend eingestellt und dann entsprechend um 90° gedreht.

Vorliegend bedeutet, wenn nicht ausdrücklich anders angegeben, der Begriff Verbindungen sowohl eine Verbindung, als auch mehrere Verbindungen.

Der Ausdruck "Alkyl" umfasst vorzugsweise geradkettige und verzweigte Alkylgruppen mit 1 bis 15 Kohlenstoffatomen, insbesondere die geradkettigen Gruppen Methyl, Ethyl, Propyl, Butyl, Pentyl, Hexyl und Heptyl. Gruppen mit 2 bis 10 Kohlenstoffatomen sind im Allgemeinen bevorzugt.

Der Ausdruck "Alkenyl" umfasst vorzugsweise geradkettige und verzweigte Alkenylgruppen mit 2 bis 15 Kohlenstoffatomen, insbesondere die geradkettigen Gruppen. Besonders bevorzugte Alkenylgruppen sind $C_2$ bis $C_7$-1E-Alkenyl, $C_4$ bis $C_7$-3E-Alkenyl, $C_5$ bis $C_7$-4-Alkenyl, $C_6$ bis $C_7$-5-Alkenyl und $C_7$-6-Alkenyl, insbesondere $C_2$ bis $C_7$-1E-Alkenyl, $C_4$ bis $C_7$-3E-Alkenyl und $C_5$ bis $C_7$-4-Alkenyl. Beispiele weiterer bevorzugter Alkenylgruppen sind Vinyl, 1 E-Propenyl, 1 E-Butenyl, 1 E-Pentenyl, 1 E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im Allgemeinen bevorzugt.

Der Ausdruck "Alkoxy" umfasst vorzugsweise geradkettige Reste der Formel $C_nH_{2n+1}$-O-, worin n 1 bis 10 bedeuten. Vorzugsweise ist n 1 bis 6. Bevorzugte Alkoxygruppen sind beispielsweise Methoxy, Ethoxy, n-Propoxy, n-Butoxy, n-Pentoxy, n-Hexoxy, n-Heptoxy, n-Octoxy, n-Nonoxy, n-Decoxy.

Der Ausdruck "Oxaalkyl" bzw. "Alkoxyalkyl" umfasst vorzugsweise geradkettige Reste der Formel $C_nH_{2n+1}$-O-$(CH_2)_m$, worin n und m jeweils unabhängig voneinander 1 bis 10 bedeuten. Vorzugsweise ist n 1 und m 1 bis 6.

Der Ausdruck "fluorierter Alkylrest" umfasst vorzugsweise ein- oder mehrfach fluorierte Reste. Perfluorierte Reste sind eingeschlossen. Besonders bevorzugt sind $CF_3$, $CH_2CF_3$, $CH_2CHF_2$, $CHF_2$, $CH_2F$, $CHFCF_3$ und $CF_2CHFCF_3$.

Der Ausdruck "fluorierter Alkoxyrest" umfasst vorzugsweise ein- oder mehrfach fluorierte Reste. Perfluorierte

Reste sind eingeschlossen. Besonders bevorzugt ist $OCF_3$.

Die erfindungsgemäßen Flüssigkristallmedien bestehen aus mehreren Verbindungen, vorzugsweise aus 3 bis 30, stärker bevorzugt aus 4 bis 20 und ganz bevorzugt aus 4 bis 16 Verbindungen. Diese Verbindungen werden auf herkömmliche Weise gemischt. In der Regel wird die gewünschte Menge der in der geringeren Menge verwendeten Verbindung in der in der größeren Menge verwendeten Verbindung gelöst. Liegt die Temperatur über dem Klärpunkt der in der höheren Konzentration verwendeten Verbindung, ist die Vervollständigung des Lösungsvorgangs besonders leicht zu beobachten. Es ist jedoch auch möglich, die Medien auf anderen üblichen Wegen, beispielsweise unter Verwendung von so genannten Vormischungen, bei denen es sich z.B. um homologe oder eutektische Mischungen von Verbindungen handeln kann, oder unter Verwendung von so genannten "Multi-Bottle"-Systemen, deren Bestandteile selbst gebrauchsfertige Mischungen sind, herzustellen.

Vorliegend bedeutet, wenn nicht ausdrücklich anders die Pluralform eines Begriffs sowohl die Singularform als auch die Pluralform, und umgekehrt.

Weitere Kombinationen der Ausführungsformen und Varianten der Erfindung gemäß der Beschreibung ergeben sich auch aus den Ansprüchen.

Weitere Abkürzungen:

| | |
|---|---|
| THF | Tetrahydrofuran |
| MTBE | Methyl-tert-butylether |
| $SiO_2$ | Kieselgel zur Chromatographie |

Die folgenden Beispiele erläutern die vorliegende Erfindung, ohne sie in irgendeiner Weise zu beschränken.

Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

**Beispiele**

Vergleichsbeispiel 1: 2-{4-[Difluor-(2,3',5'-trifluor-4'-trifluormethyl-biphenyl-4-yloxy)-methyl]-3,5-difluor-phenyl}-5-propyl-tetrahydropyran ("AUQGU-3-T")

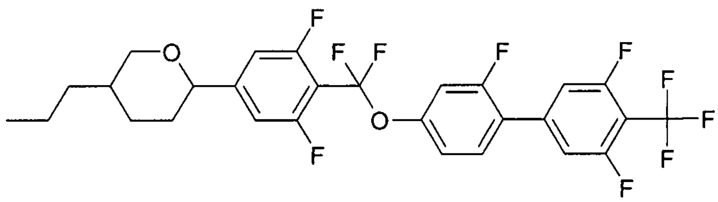

Die Verbindung wird hergestellt wie nachfolgend beschrieben:

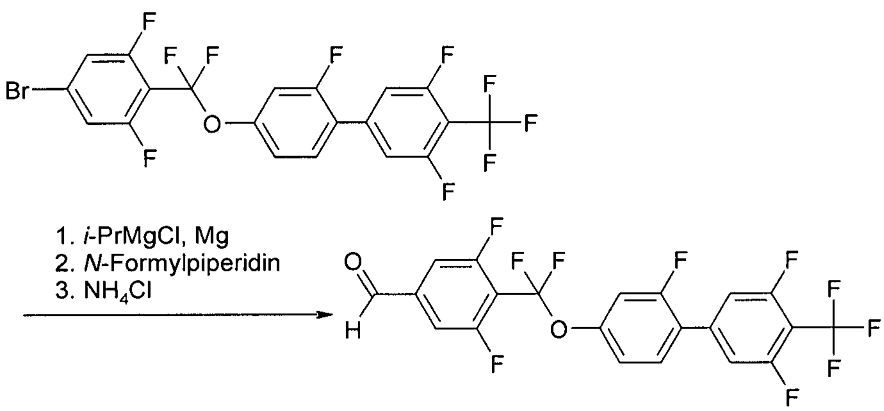

100 mg (4 mmol) Magnesiumspäne werden in 150 ml THF vorgelegt, und 30,0 ml (60,0 mmol) *i*-PrMgCl (2M Lsg. in THF) werden zugegeben. Nach 30 min wird eine Lösung von 22,0 g (41,3 mmol) 4'-[(4-Brom-2,6-difluorphenyl)-difluormethoxy]-3,5,2'-trifluor-4-trifluormethyl-biphenyl in 100 ml THF zugetropft. Nach beendeter Zugabe wird 2 h bei Raumtemperatur gerührt. 7,0 ml (63,1 mmol) *N*-Formylpiperidin in 50 ml THF werden zugegeben, und die Mischung wird 19 h gerührt. Der Ansatz wird mit gesättigter Ammoniumchloridlösung versetzt und mehrfach mit MTBE extrahiert. Die vereinigten organischen Phasen werden mit gesättigter Natriumchloridlösung gewaschen, und die Lösung wird mit Natriumsulfat getrocknet. Das nach Entfernen der Lösungsmittel verbleibende Rohprodukt wird säulenchromatographisch ($SiO_2$, *n*-Heptan : Toluol = 1 : 1) gereinigt. Auf diese Weise wird 4-[Difluor-(2,3',5'-trifluor-4'-trifluormethyl-biphenyl-4-yloxy)-methyl]-3,5-difluor-benzaldehyd als gelblicher Feststoff erhalten.

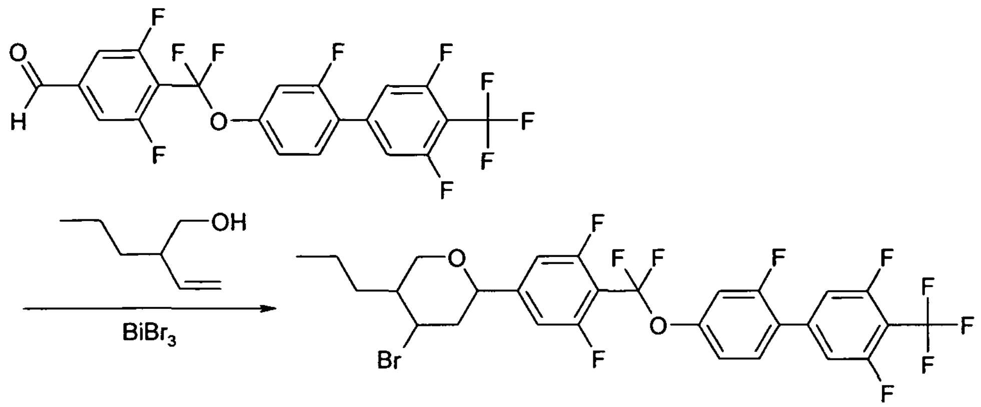


9,0 g (18,5 mmol) 4-[Difluor-(2,3',5'-trifluor-4'-trifluormethyl-biphenyl-4-yloxy)-methyl]-3,5-difluor-benzaldehyd werden zusammen mit 2,20 g (19,3 mmol) 2-Vinyl-pentan-1-ol in 120 ml Dichlormethan vorgelegt, und 4,40 g (9,61 mmol) Bismuth(III)-bromid werden portionsweise zugegeben. Der Ansatz wird 22 h gerührt, und die Mischung wird absorptiv ($SiO_2$, Dichlormethan) filtriert. Das Filtrat wird vollständig konzentriert, und das Rohprodukt wird säulenchromatographisch ($SiO_2$, *n*-Heptan : Toluol = 1 : 1) gereinigt. 4-Brom-2-{4-[difluor-(2,3',5'-trifluor-4'-trifluormethyl-biphenyl-4-yloxy)-methyl]-3,5-difluor-phenyl}-5-propyl-tetrahydropyran wird als farbloser Feststoff erhalten. Die Mischung zweier Stereoisomere wird für die nächste Umsetzung verwendet.

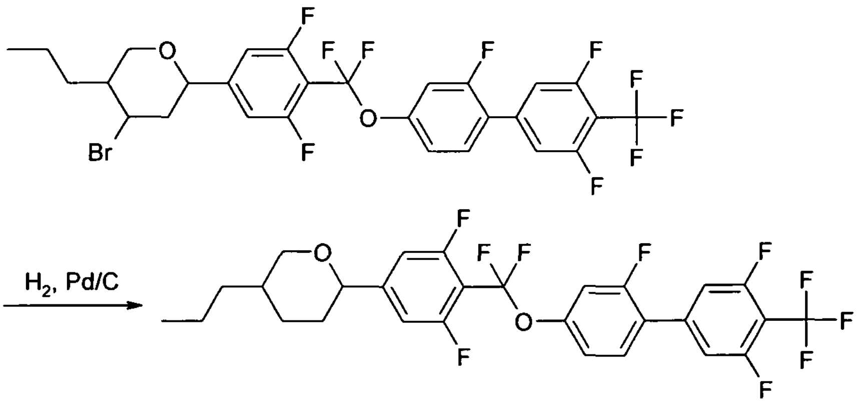


10,0 g (14,7 mmol) 4-Brom-2-{4-[difluor-(2,3',5'-trifluor-4'-trifluormethyl-biphenyl-4-yloxy)-methyl]-3,5-difluor-phenyl}-5-propyl-tetrahydropyran werden in THF gelöst und in Gegenwart von Triethylamin und Pd/C 50 h bei 5,8 bar Wasserstoffdruck und 50 °C hydriert. Die Lösung wird filtriert und vollständig konzentriert. Das Rohprodukt wird säulenchromatographisch ($SiO_2$, *n*-Heptan : Toluol = 1 : 1) gereinigt. Die weitere Reinigung erfolgt durch Umkristallisieren aus Isopropanol. Auf diese Weise wird *trans*-2-{4-[Difluor-(2,3',5'-trifluor-4'-trifluormethyl-biphenyl-4-yloxy)-methyl]-3,5-difluor-phenyl}-5-propyl-tetrahydropyran ("AUQGU-3-T") als farbloser Feststoff mit einem Schmelzpunkt von 95 °C erhalten.
K 95 N (81) I
$\Delta\varepsilon = 37$
$\Delta n = 0{,}131$
$\gamma_1 = 752$ mPa·s
$\Delta\varepsilon \cdot \Delta n = 4{,}9$
**$^1$H-NMR** (300 MHz, $CHCl_3$): $\delta$ = 7,44-7,36 (m, 1H, $H_{arom.}$), 7,23-7,12 (m, 4H, $H_{arom.}$), 6,99 (d, 2H, J = 10,8 Hz, $H_{arom}$),

4,26 (dd, 1H, J = 11,3 Hz, J = 1,8 Hz, $H_{pyranyl.}$), 4,09 (ddd, 1 H, J = 11,3 Hz, J = 4,5 Hz, J = 1,8 Hz, $H_{pyranyl.}$), 3,19 (t, 1 H, J = 11,3 Hz, $H_{pyranyl.}$), 2,05-1,96 (m, 1H, $H_{aliph.}$), 1,93-1,85 (m, 1H, $H_{aliph.}$), 1,73-1,58 (m, 1H, $H_{aliph.}$), 1,55-1,07 (m, 6H, $H_{arom.}$), 0,92 (t, 3H, J = 7,4 Hz, -$CH_2CH_2$**$CH_3$**).
**$^{19}$F-NMR** (282 MHz, $CHCl_3$): $\delta$ = -56,3 (t, 3F, J = 21,9 Hz, -$CF_3$), -60,9 (t, 2F, J = 25,6 Hz, -$OCF_2$-), -110,5 bis -110,7 (m, 4F, $F_{arom}$.), -113,6 (s, 1F, $F_{arom.}$).
**MS (EI):** m/z(%) = 580 (3, $M^+$), 561 (3, $[M - F]^+$), 289 (100).

Beispiel 2: 2-{4-[Difluor-(2,3',5'-trifluor-4'-trifluormethoxy-biphenyl-4-yloxy)-methyl]-3,5-difluorphenyl}-5-propyl-[1,3]dioxan ("DUQGU-3-OT")

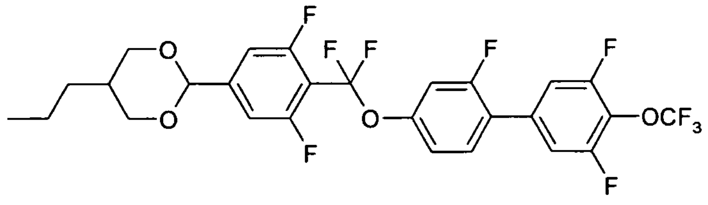

Die erfindungsgemäße Verbindung wird hergestellt wie nachfolgend beschrieben:

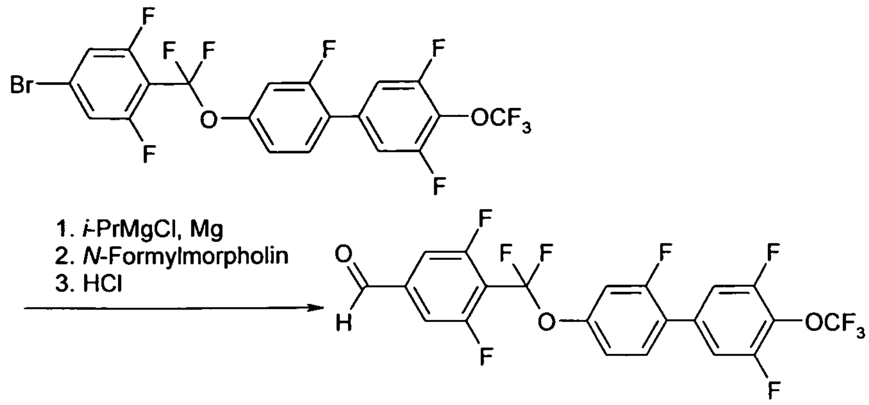


90 mg (3,7 mmol) Magnesiumspäne werden in 200 ml THF vorgelegt, und 26,0 ml (52,0 mmol) *i*-PrMgCl (2M Lsg. in THF) werden zugegeben. Nach 30 min wird eine Lösung von 19,0 g (34,6 mmol) 4'-[(4-Brom-2,6-difluor-phenyl)-difluormethoxy]-3,5,2'-trifluor-4-trifluormethoxy-biphenyl in 50 ml THF zugetropft. Nach beendeter Zugabe wird 4 h bei Raumtemperatur gerührt. 5,8 ml (52,3 mmol) *N*-Formylpiperidin in 30 ml THF werden zugegeben, und die Mischung wird 21 h gerührt. Der Ansatz wird mit verd. Salzsäure versetzt und mehrfach mit MTBE extrahiert. Die vereinigten organischen Phasen werden mit gesättigter Natriumhydrogencarbonatlösung gewaschen, und die Lösung wird mit Natriumsulfat getrocknet. Das nach Entfernen der Lösungsmittel verbleibende Rohprodukt wird säulenchromatographisch ($SiO_2$, *n*-Heptan : Toluol = 1 : 1) gereinigt. Auf diese Weise wird 4-[Difluor-(2,3',5'-trifluor-4'-trifluormethoxy-biphenyl-4-yloxy)-methyl]-3,5-difluor-benzaldehyd als gelblicher Feststoff erhalten.

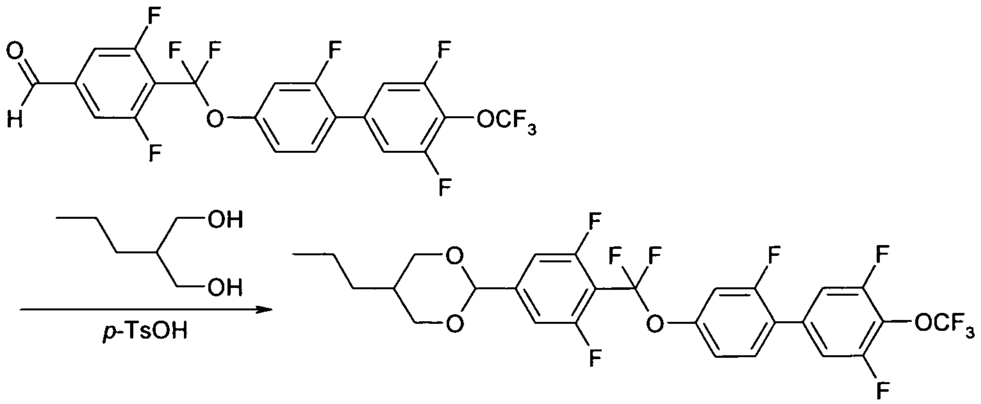


3,50 g (7,02 mmol) 4-[Difluor-(2,3',5'-trifluor-4'-trifluormethoxy-biphenyl-4-yloxy)-methyl]-3,5-difluor-benzaldehyd werden zusammen mit 850 mg 2-n-Propyl-propan-1,3-diol und 30 mg *p*-Toluolsulfonsäure-Monohydrat in 100 ml Toluol 3 h am Wasserabscheider refluxiert. Nach dem Abkühlen wird der Ansatz mit Wasser und gesättigter Natriumhydrogencarbonatlösung gewaschen. Die Lösung wird mit Natriumsulfat getrocknet und vollständig konzentriert. Das Rohprodukt wird säulenchromatographisch ($SiO_2$, Toluol) gereinigt. Die weitere Reinigung erfolgt durch Umkristallisation aus Ethanol und *n*-Heptan. 2-{4-[Difluor-(2,3',5'-trifluor-4'-trifluormethoxy-biphenyl-4-yloxy)-methyl]-3,5-difluorphenyl}-5-propyl-[1,3]dioxan wird als farbloser Feststoff mit einem Schmelzpunkt von 61 °C erhalten.

K 61 N 93 I

$\Delta\varepsilon = 36$

$\Delta n = 0,129$

$\gamma_1 = 634$ mPa·s

$\Delta\varepsilon \cdot \Delta n = 4,7$

**$^1$H-NMR** (300 MHz, $CHCl_3$): $\delta$ = 7,40-7,33 (m, 1 H, $H_{arom.}$), 7,23-7,11 (m, 6H, $H_{arom.}$), 5,37 (s, 1H, $H_{dioxanyl.}$), 4,24 (ddd, 2H, J = 11,0 Hz, J = 4,6 Hz, J = 1,5 Hz, $H_{dioxanyl.}$), 3,52 (dt, 2, H, J = 10,8 Hz, J = 1,5 Hz, $H_{dioxanyl.}$), 2,20-2,05 (m, 1H, $H_{aliphat.}$), 1,41-1,27 (m, 2H, $H_{aliphat.}$), 1,14-1,04 (m, 2H, $H_{aliphat.}$), 0,98 (t, 3H, J = 7,3 Hz, $-CH_2CH_2\mathbf{CH_3}$).

**$^{19}$F-NMR** (282 MHz, $CHCl_3$): $\delta$ = -59,7 (t, 3F, J = 7,3 Hz, $-OCF_3$), -60,9 (t, 2F, J = 26,8 Hz, $-OCF_2-$), -110,0 (dt, 2F, J = 26,8 Hz, J = 9,2 Hz, $F_{arom.}$), -114,0 (t, 1 F, J = 9,2 Hz, $F_{arom.}$), -124,5 bis -124,6 (m, 2F, $F_{arom.}$).

**MS (EI):** m/z(%) = 598 (12, $M^+$), 579 (2, $[M - F]^+$), 291 (100).

Analog zu Beispiel 1 werden die Vergleichsbeispielverbindungen 3 bis 5 hergestellt. Die spektroskopischen Daten (NMR, MS) entsprechen jeweils den Strukturen.

| | | |
|---|---|---|
| 3 | 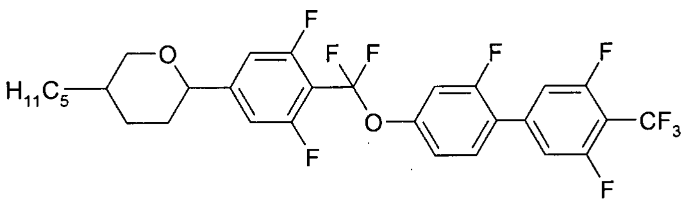<br>"AUQGU-5-T" | K 74 SmA (39) N 89 I<br>$\Delta\varepsilon = 38$<br>$\Delta n = 0,130$<br>$\gamma1 = 1194$ mPa·s<br>$\Delta\varepsilon \cdot \Delta n = 5,0$ |
| 4 | 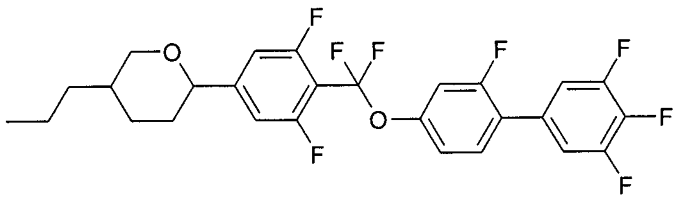<br>"AUQGU-3-F" | Tg -42 K 49 N 82 I<br>$\Delta\varepsilon = 27$<br>$\Delta n = 0,129$<br>$\gamma1 = 459$ mPa·s<br>$\Delta\varepsilon \cdot \Delta n = 3,5$ |

(fortgesetzt)

| | | |
|---|---|---|
| 5 | 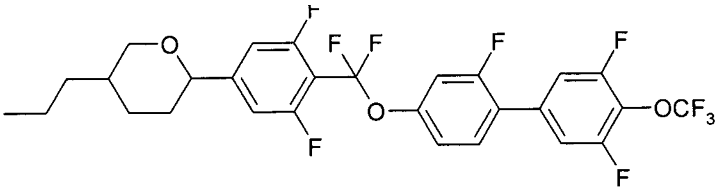 "AUQGU-3-OT" | Tg -46 K 61 SmA (12) N 102 I<br>$\Delta\varepsilon$ = 30<br>$\Delta n$ = 0,125<br>$\gamma_1$ = 615 mPa·s<br>$\Delta\varepsilon \cdot \Delta n$ = 3,8 |

Analog zu Beispiel 2 werden die Vergleichsbeispielverbindung 6 und Beispielverbindung 7 hergestellt. Die spektroskopischen Daten (NMR, MS) entsprechen jeweils den Strukturen.

| | | |
|---|---|---|
| 6 | 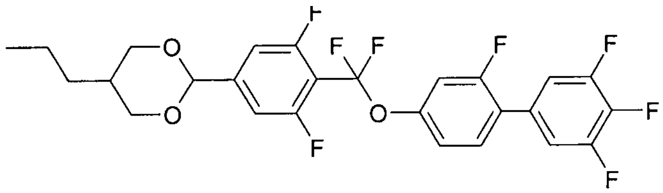 "DUQGU-3-F" | K 61 N 79<br>$\Delta\varepsilon$ = 35<br>$\Delta n$ = 0,129<br>$\gamma_1$ = 436<br>$\Delta\varepsilon \cdot \Delta n$ = 4,5 |
| 7 | 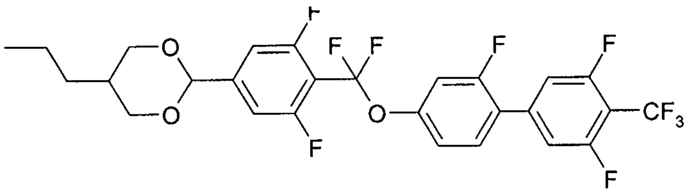 "DUQGU-3-T" | K 96 I<br>$\Delta\varepsilon$ = 40<br>$\Delta n$ = 0,1314<br>$\gamma_1$ = 744<br>$\Delta\varepsilon \cdot \Delta n$ = 5,3 |

**Mischungsbeispiele**

Die folgenden Akronyme werden verwendet, um die Komponenten der flüssigkristallinen Grundmischung (Host) zu beschreiben. Der Zähler n nimmt einen Wert von 1 bis 9 an. Die Verbindungen eignen sich für die Herstellung von erfindungsgemäßen flüssigkristallinen Medien.

Tabelle A: Weitere Akronyme für LC-Komponenten

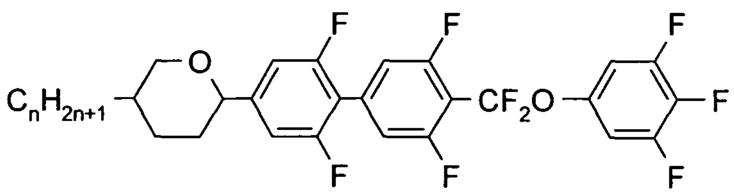

**AUUQU-n-F**

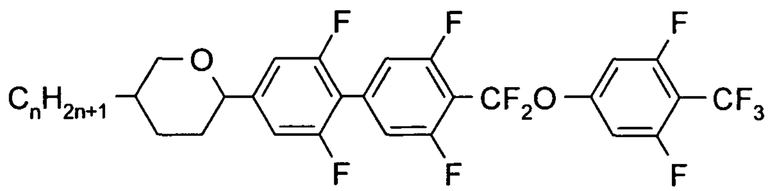

**AUUQU-n-T**

(fortgesetzt)

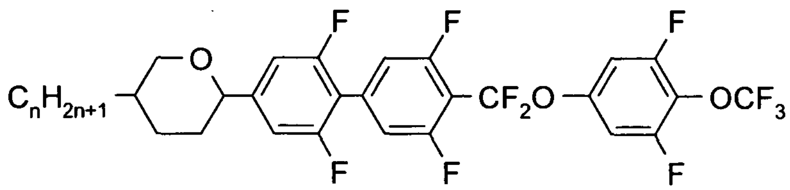

**AUUQU-n-OT**

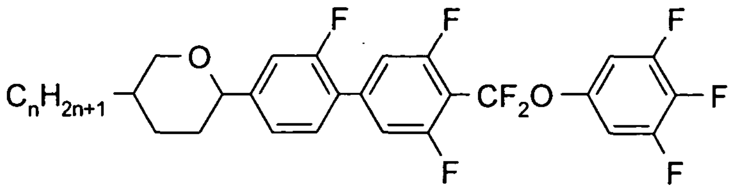

**AGUQU-n-F**

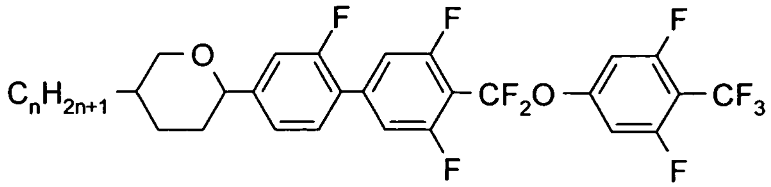

**AGUQU-n-T**

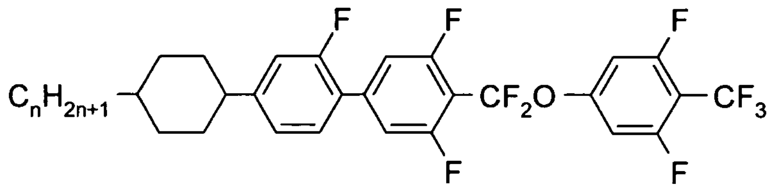

**CGUQU-n-T**

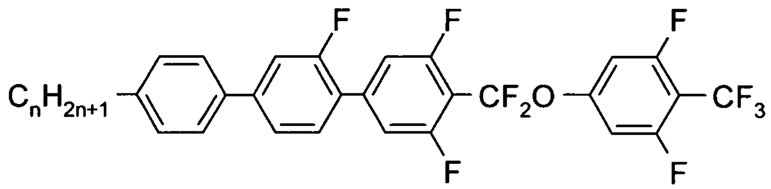

**PGUQU-n-T**

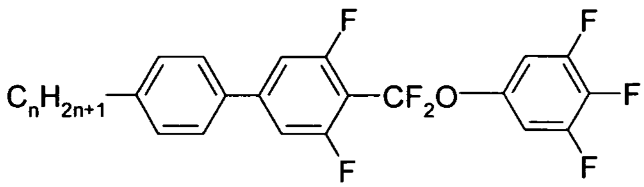

**PUQU-n-F**

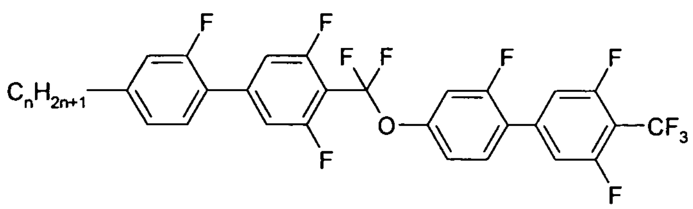

**GUQGU-n-T**

Die folgenden Monomere werden vorzugsweise verwendet:

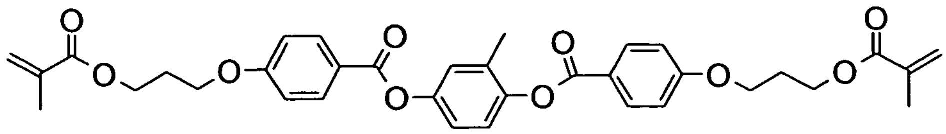

**RM220**

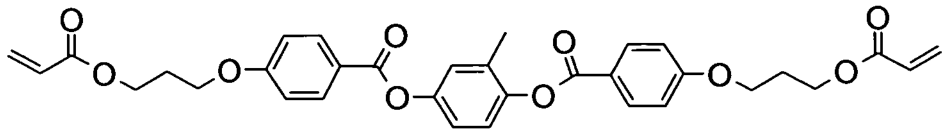

**RM257**

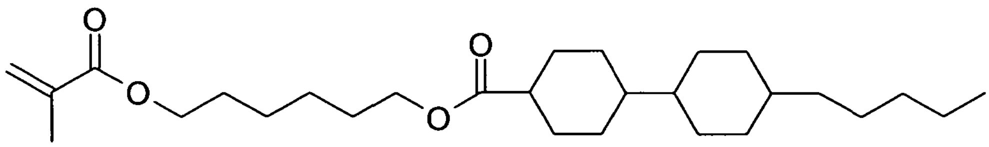

**RM-2**

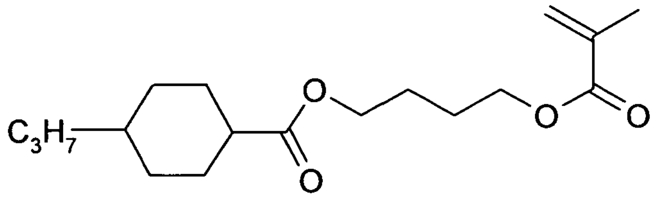

**RM-3**

RM220 hat die Phasensequenz K 82,5 N 97 I.
RM257 hat die Phasensequenz K 66 N 127 I.

Die folgenden Zusatzstoffe werden vorzugsweise verwendet (DP: chirales Dopant, IN: Polymerisationsinitiator):

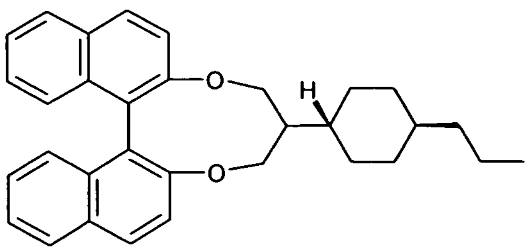

**DP-1**

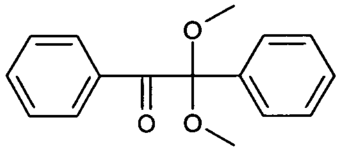

**IN-1** (Ciba ® Irgacure ® 651)

Weitere chirale Dopants und Polymerisationsinitiatoren für LC-Mischungen sind dem Fachmann bekannt und werden hier ausdrücklich erwähnt.

Die Medien werden vor der Polymerisation wie beschrieben charakterisiert. Darauf werden die RM-Komponenten durch einmalige Bestrahlung (180 s) in der blauen Phase polymerisiert, und die erhaltenen Medien werden erneut charakterisiert.

Beschreibung der Polymerisation

Vor der Polymerisation einer Probe werden die Phaseneigenschaften des Mediums in einer Testzelle von ca. 10 Mikrometer Dicke und einer Fläche von 2x2,5 cm festgestellt. Die Füllung erfolg durch Kapillarwirkung bei einer Temperatur von 75 °C. Die Messung des unpolymerisierten Mediums erfolgt unter einem Polarisationsmikroskop mit Heiztisch bei einem Temperaturverlauf von 1 °C/min.
Die Polymerisation der Medien wird durch Bestrahlung mit einer UV-Lampe (Hönle, Bluepoint 2.1, 365 nm Interferenzfilter) mit einer effektiven Leistung von ca. 1,5 $mW/cm^2$ für 180 Sekunden durchgeführt. Die Polymerisation erfolgt direkt in der elektrooptischen Testzelle. Die Polymerisation erfolgt anfangs bei einer Temperatur, in der das Medium in der blauen Phase I (BP-I) vorliegt. Die Polymerisation erfolgt in mehreren Teilschritten, die nach und nach zu einer vollständigen Polymerisation führen. Der Temperaturbereich der blauen Phase ändert sich in der Regel während der Polymerisation. Zwischen jedem Teilschritt wird daher die Temperatur so angepasst, dass das Medium nach wie vor in der blauen Phase vorliegt. In der Praxis kann dies so erfolgen, dass nach jedem Bestrahlungsvorgang von ca. 5 s oder länger die Probe unter dem Polarisationsmikroskop beobachtet wird. Wird die Probe dunkler, so deutet dies auf einen Übergang in die isotrope Phase hin. Die Temperatur für den nächsten Teilschritt wird entsprechend verringert. Die gesamte Bestrahlungszeit, die zu der maximalen Stabilisierung führt, beträgt typischerweise 180 s bei der angegebenen Bestrahlungsleistung. Weitere Polymerisationen können nach einem optimierten Bestrahlungs-Temperatur-Programm durchgeführt werden. Alternativ kann die Polymerisation auch in einem einzigen Bestrahlungsschritt durchgeführt werden, insbesondere dann, wenn schon vor der Polymerisation eine ausreichend breite blaue Phase vorliegt.

Elektrooptische Charakterisierung

Nach der oben beschriebenen Polymerisation und Stabilisierung der blauen Phase wird die Phasenbreite der blauen Phase bestimmt. Die elektrooptische Charakterisierung erfolgt anschließend bei verschiedenen Temperaturen innerhalb und ggf. auch außerhalb dieses Bereichs.

Die verwendeten Testzellen sind auf einer Seite mit Interdigitalelektroden auf der Zellenoberfläche ausgestattet. Der Zellspalt, der Elektrodenabstand und die Elektrodenbreite betragen typischerweise jeweils 1 bis 10 Mikrometer und sind bevorzugt von gleicher Größe. Dieses einheitliche Maß wird nachfolgend als Spaltbreite bezeichnet. Die mit Elektroden belegte Fläche beträgt ca. 0,4 $cm^2$. Die Testzellen besitzen keine Orientierungsschicht ('alignment layer'). Die Zelle befindet sich für die elektrooptische Charakterisierung zwischen gekreuzten Polarisationsfiltern, wobei die Längsrichtung der Elektroden einen Winkel von 45° mit den Achsen des Polarisationsfilters einnimmt. Die Messung erfolgt mit einem DMS301 (Autronic-Melchers) im rechten Winkel zur Zellebene oder mittels einer hochempfindlichen Kamera am Polarisationsmikroskop. Im spannungslosen Zustand ergibt die beschriebene Anordnung ein im Wesentlichen dunkles Bild (Definition 0 % Transmission).

Zuerst werden die charakteristischen Betriebsspannungen und dann die Schaltzeiten an der Testzelle gemessen. Die Betriebsspannung an den Zellelektroden wird in Form von Rechteckspannung mit alternierendem Vorzeichen (Frequenz 100 Hz) und variabler Amplitude wie im Folgenden beschrieben angelegt.

Die Transmission im spannungslosen Zustand wird als 0 % festgelegt. Während die Betriebspannung erhöht wird, wird die Transmission gemessen. Das Erreichen des Maximalwerts von ca. 100 % Intensität legt die charakteristische Größe der Betriebsspannung $V_{100}$ fest. Gleichermaßen wird die charakteristische Spannung $V_{10}$ bei 10 % der maximalen Transmission bestimmt. Diese Werte werden optional bei verschiedenen Temperaturen im Bereich der blauen Phase gemessen, jedenfalls bei Raumtemperatur (20°C).

Am unteren Ende des Temperaturbereichs der blauen Phase werden relativ hohe charakteristische Betriebsspannungen $V_{100}$ beobachtet. Am oberen Ende des Temperaturbereichs (Nähe zum Klärpunkt) steigt der Wert von $V_{100}$ stark an. Im Bereich der minimalen Betriebsspannung steigt $V_{100}$ in der Regel nur langsam mit der Temperatur. Dieser Temperaturbereich, begrenzt durch $T_1$ und $T_2$ wird als nutzbarer, flacher Temperaturbereich (FB) bezeichnet. Die Breite dieses "Flachbereichs" (FB) beträgt ($T_2$ - $T_1$) und wird als Breite des Flachbereichs (BFB) (engl. 'flat range') bezeichnet. Die genauen Werte von $T_1$ und $T_2$ werden durch die Schnittpunkte von Tangenten an den flachen Kurvenabschnitt FB und die benachbarten steilen Kurvenabschnitte im $V_{100}$-Temperatur-Diagramm ermittelt.

Im zweiten Teil der Messung werden die Schaltzeiten beim Ein- und Ausschalten ermittelt ($\tau_{on}$, $\tau_{off}$). Die Schaltzeit $\tau_{on}$ ist definiert durch die Zeit bis zum Erreichen von 90 % Intensität nach dem Anlegen einer Spannung der Höhe von $V_{100}$ bei der gewählten Temperatur. Die Schaltzeit $\tau_{off}$ ist definiert durch die Zeit bis zur Abnahme um 90 % ausgehend

von maximaler Intensität bei $V_{100}$ nach dem Erniedrigen der Spannung auf 0 V. Auch die Schaltzeit wird bei verschiedenen Temperaturen im Bereich der blauen Phase ermittelt.

Als weitere Charakterisierung kann bei einer Temperatur innerhalb FB die Transmission bei kontinuierlich veränderter Betriebsspannung zwischen 0 V und $V_{100}$ gemessen werden. Bei Vergleich der Kurven für zunehmende und für abnehmende Betriebsspannung kann eine Hysterese auftreten. Die Differenz der Transmissionen bei $0{,}5 \cdot V_{100}$ bzw. die Differenz der Spannungen bei 50 % Transmission sind beispielsweise charakteristische Hysteresewerte und werden als $\Delta T_{50}$ respektive $\Delta V_{50}$ bezeichnet.

Als weitere Kenngröße kann das Verhältnis der Transmission im spannungslosen Zustand vor und nach Durchlaufen eines Schaltzyklusses gemessen werden. Dieses Transmissionsverhältnis wird als "Memory-Effekt" bezeichnet. Der Wert des Memory-Effekts liegt im Idealzustand bei 1,0. Werte über 1 bedeuten, dass ein gewisser Memory-Effekt in Form einer zu hohen Resttransmission nach dem Ein- und Ausschalten der Zelle vorliegt. Auch dieser Wert wird im Arbeitsbereich der blauen Phase (FB) ermittelt.

Die Messwerte, soweit nicht anders angegeben, werden bei 20 °C ermittelt.

Mischungsbeispiele

**Mischungsvergleichsbeispiel 1** (Hostmischung)

| Komponente | Gew. % |
|---|---|
| AUUQU-2-F | 10 |
| AUUQU-3-F | 8 |
| AUUQU-5-F | 6 |
| AUUQU-3-T | 8 |
| AUQGU-3-F | 8 |
| PUZU-2-F | 5 |
| PUZU-3-F | 5 |
| PUZU-5-F | 5 |
| AGUQU-3-F | 4 |
| AUUQU-3-N | 5 |
| GUQGU-2-T | 9 |
| GUQGU-3-T | 9 |
| GUQGU-4-T | 9 |
| GUQGU-5-T | 9 |
| Klärpunkt: 76 °C | |

**Mischungsvergleichsbeispiel 2** (Hostmischung)

| Komponente | Gew. % |
|---|---|
| AUUQU-2-F | 10 |
| AUUQU-3-F | 8 |
| AUUQU-5-F | 6 |
| AUUQU-3-T | 8 |
| AUQGU-3-OT | 8 |
| PUZU-2-F | 5 |
| PUZU-3-F | 5 |

(fortgesetzt)

| Komponente | Gew. % |
|---|---|
| PUZU-5-F | 5 |
| AGUQU-3-F | 4 |
| AUUQU-3-N | 5 |
| GUQGU-2-T | 9 |
| GUQGU-3-T | 9 |
| GUQGU-4-T | 9 |
| GUQGU-5-T | 9 |

**Mischungsvergleichsbeispiel 3** (Hostmischung)

| Komponente | Gew. % |
|---|---|
| AUUQU-2-F | 10 |
| AUUQU-3-F | 8 |
| AUUQU-5-F | 6 |
| AUUQU-3-T | 8 |
| AUQGU-3-T | 8 |
| PUZU-2-F | 5 |
| PUZU-3-F | 5 |
| PUZU-5-F | 5 |
| AGUQU-3-F | 4 |
| AUUQU-3-N | 5 |
| GUQGU-2-T | 9 |
| GUQGU-3-T | 9 |
| GUQGU-4-T | 9 |
| GUQGU-5-T | 9 |

**Mischungsvergleichsbeispiel 4** (Hostmischung)

| Komponente | Gew. % |
|---|---|
| AUUQU-2-F | 10 |
| AUUQU-3-F | 8 |
| AUUQU-5-F | 6 |
| AUUQU-3-T | 8 |
| DUQGU-3-F | 8 |
| PUZU-2-F | 5 |
| PUZU-3-F | 5 |
| PUZU-5-F | 5 |
| AGUQU-3-F | 4 |
| AUUQU-3-N | 5 |

(fortgesetzt)

| Komponente | Gew. % |
|---|---|
| GUQGU-2-T | 9 |
| GUQGU-3-T | 9 |
| GUQGU-4-T | 9 |
| GUQGU-5-T | 9 |

**Mischungsbeispiel 5** (Hostmischung)

| Komponente | Gew. % |
|---|---|
| AUUQU-2-F | 10 |
| AUUQU-3-F | 8 |
| AUUQU-5-F | 6 |
| AUUQU-3-T | 8 |
| DUQGU-3-OT | 8 |
| PUZU-2-F | 5 |
| PUZU-3-F | 5 |
| PUZU-5-F | 5 |
| AGUQU-3-F | 4 |
| AUUQU-3-N | 5 |
| GUQGU-2-T | 9 |
| GUQGU-3-T | 9 |
| GUQGU-4-T | 9 |
| GUQGU-5-T | 9 |

**Mischungsbeispiel 6** (Hostmischung)

| Komponente | Gew. % |
|---|---|
| AUUQU-2-F | 10 |
| AUUQU-3-F | 8 |
| AUUQU-5-F | 6 |
| AUUQU-3-T | 8 |
| DUQGU-3-T | 8 |
| PUZU-2-F | 5 |
| PUZU-3-F | 5 |
| PUZU-5-F | 5 |
| AGUQU-3-F | 4 |
| AUUQU-3-N | 5 |
| GUQGU-2-T | 9 |
| GUQGU-3-T | 9 |
| GUQGU-4-T | 9 |

(fortgesetzt)

| Komponente | Gew. % |
|---|---|
| GUQGU-5-T | 9 |

**Mischungsbeispiel** 7

Eine typische polymerstabilisierbare Mischung setzt sich zusammen gemäß der Tabelle:

| Komponente | Gew. % |
|---|---|
| Hostmischung (5-6) | 85 |
| IN-1 | 0,2 |
| monoreaktives Mesogen RM-2 / RM-3 | 5 |
| direaktives Mesogen RM220/RM257 | 6 |
| chirales Dopant DP-1 | 3,8 |

Die polymerisierbare Mischung wird bei einer Temperatur von ca. 30-50 °C am unteren Ende des Temperaturbereichs der blauen Phase in einem einzigen Bestrahlungsschritt polymerisiert (Details vgl. oben).

Die polymerstabilisierten flüssigkristallinen Medien zeigen über einen breiten Temperaturbereich eine blaue Phase.

**Patentansprüche**

**1.** Verbindungen der Formel I

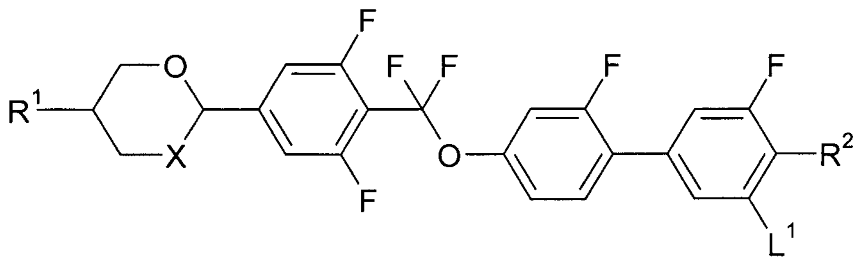

I

worin

$L^1$ F,
X O,
$R^1$ einen unsubstituierten Alkylrest mit 1 bis 15 C-Atomen, wobei in diesem Rest auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)- oder -O- so ersetzt sein können, dass O-Atome nicht direkt miteinander verknüpft sind,

und

$R^2$ $CF_3$ oder $OCF_3$

bedeuten.

**2.** Verbindungen nach Anspruch 1, **dadurch gekennzeichnet, dass** $R^2$ ein Gruppe $CF_3$ bedeutet.

**3.** Verbindungen nach einem oder mehreren der Ansprüche 1 oder 2, **dadurch gekennzeichnet, dass** $R^1$ einen Alkylrest mit 1 bis 9 C-Atomen oder einen Alkenylrest mit 2 bis 9 C-Atomen bedeutet.

4. Verbindungen nach einem oder mehreren der Ansprüche 1 bis 3, **dadurch gekennzeichnet, dass** $R^1$ einen geradkettigen Alkylrest mit 3 C-Atomen bedeutet.

5. Verfahren zur Herstellung von Verbindungen der Formel I nach einem oder mehreren der Ansprüche 1 bis 4 umfassend einen Reaktionsschritt wobei zwei Edukte der Formeln A und B:

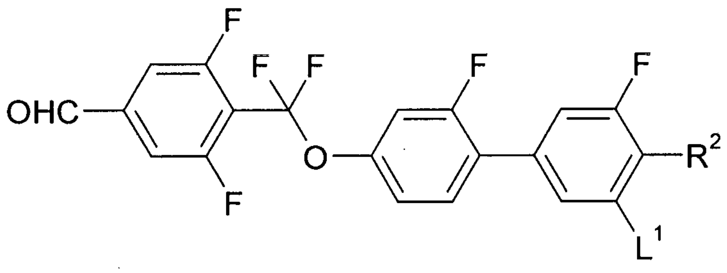

A

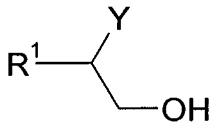

B

worin $R^1$, $R^2$ und $L^1$ wie für Formel I definiert sind, und
Y einen Rest der Formel $-CH_2-OH$ bedeutet,

unter geeigneten Reaktionsbedingungen umgesetzt werden.

6. Flüssigkristallines Medium, **dadurch gekennzeichnet, dass** es eine oder mehrere Verbindungen der Formel I nach einem oder mehreren der Ansprüche 1 bis 4 enthält.

7. Flüssigkristallines Medium nach Anspruch 6, **dadurch gekennzeichnet, dass** es zusätzlich eine oder mehrere Verbindungen ausgewählt aus den Verbindungen der Formel II und III enthält:

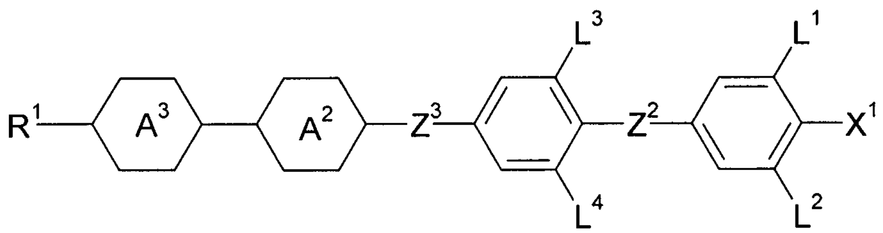

II

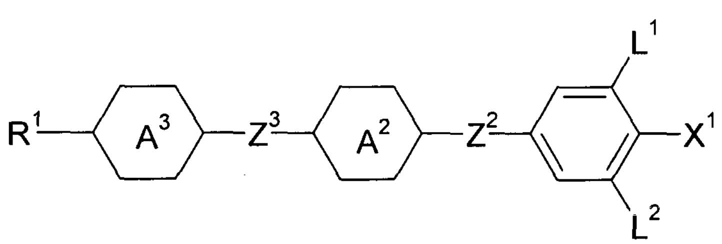

III

worin

$R^1$ unabhängig voneinander einen unsubstituierten Alkylrest mit 1 bis 15 C-Atomen, wobei in diesem Rest auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)- oder -O- so ersetzt sein können, dass O-Atome nicht direkt miteinander verknüpft sind,
$A^2$, $A^3$ unabhängig voneinander

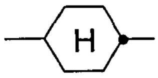, 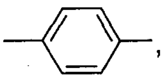, 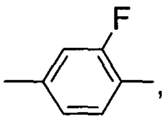, 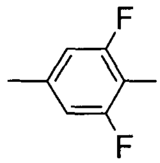,

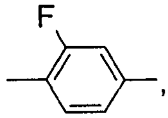, 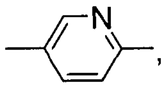, 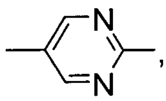, 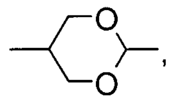,

$Z^2$, $Z^3$ unabhängig voneinander eine Einfachbindung, $CF_2O$, $CH_2CH_2$, $CF_2CH_2$, $CF_2CF_2$, CFHCFH, $CFHCH_2$, (CO)O, $CH_2O$, C≡C, CH=CH, CF=CH, CF=CF; wobei unsymmetrische Bindeglieder in beide möglichen Richtungen orientiert sein können,

$X^1$ F, Cl, CN, oder

Alkyl, Alkenyl, Alkenyloxy, Alkoxyalkyl oder Alkoxy mit 1 bis 3 C-Atomen, welches durch F ein- oder mehrfach substituiert ist, und

$L^1$ bis $L^4$ H oder F,

bedeuten.

8. Verwendung der Verbindungen der Formel I nach einem oder mehreren der Ansprüche 1 bis 4 in einem flüssigkristallinen Medium oder in einer elektrooptischen Anzeige.

9. Elektrooptische Anzeigevorrichtung enthaltend ein flüssigkristallines Medium nach Anspruch 6 oder 7.

10. Elektrooptische Anzeigevorrichtung nach Anspruch 9, **dadurch gekennzeichnet, dass** sie ganz oder teilweise im Bereich der flüssigkristallinen blauen Phase arbeitet.

## Claims

1. Compounds of the formula I

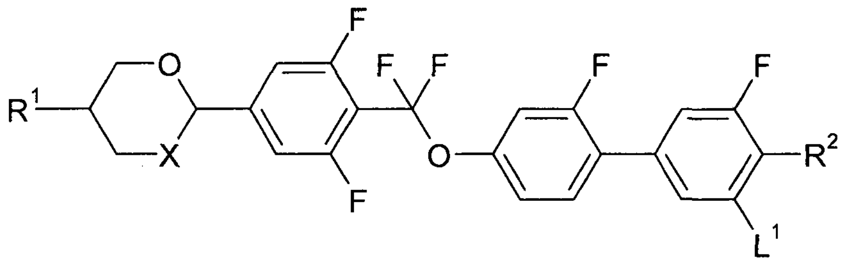 I

in which

$L^1$ denotes F,

X denotes O,

$R^1$ denotes an unsubstituted alkyl radical having 1 to 15 C atoms, where, in addition, one or more $CH_2$ groups in this radical may each be replaced, independently of one another, by -C≡C-, -CH=CH-, -CF=CF-, -CF=CH-, -CH=CF-, -(CO)O-, -O(CO)-, -(CO)- or -O- in such a way that O atoms are not linked directly to one another,

and

$R^2$ denotes $CF_3$ or $OCF_3$.

2. Compounds according to Claim 1, **characterised in that** $R^2$ denotes a $CF_3$ group.

3. Compounds according to one or more of Claims 1 or 2, **characterised in that**

$R^1$ denotes an alkyl radical having 1 to 9 C atoms or an alkenyl radical having 2 to 9 C atoms.

4. Compounds according to one or more of Claims 1 to 3, **characterised in that** $R^1$ denotes a straight-chain alkyl radical having 3 C atoms.

5. Process for the preparation of compounds of the formula I according to one or more of Claims 1 to 4, including a reaction step in which two starting materials of the formulae A and B:

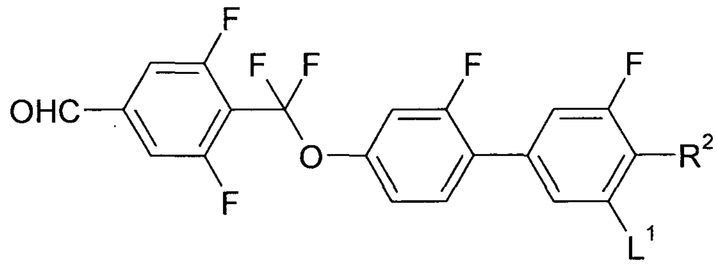

A

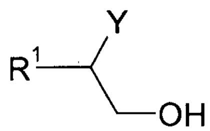

B

in which $R^1$, $R^2$ and $L^1$ are as defined for formula I, and
Y denotes a radical of the formula $-CH_2-OH$,

are reacted under suitable reaction conditions.

6. Liquid-crystalline medium, **characterised in that** it comprises one or more compounds of the formula I according to one or more of Claims 1 to 4.

7. Liquid-crystalline medium according to Claim 6, **characterised in that** it additionally comprises one or more compounds selected from the compounds of the formulae II and III:

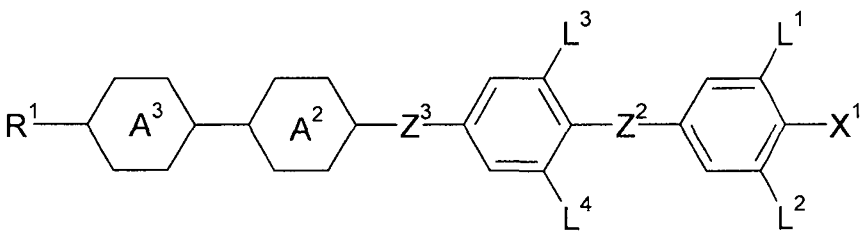

II

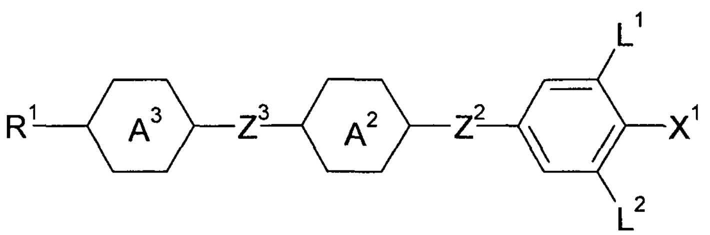

III

in which

$R^1$, independently of one another, denotes an unsubstituted alkyl radical having 1 to 15 C atoms, where, in addition, one or more $CH_2$ groups in this radical may each be replaced, independently of one another, by -C≡C-, -CH=CH-, -CF=CF-, -CF=CH-, -CH=CF-, -(CO)O-, -O(CO)-, -(CO)- or -O- in such a way that O atoms are not

linked directly to one another,

$A^2$, $A^3$, independently of one another, denote

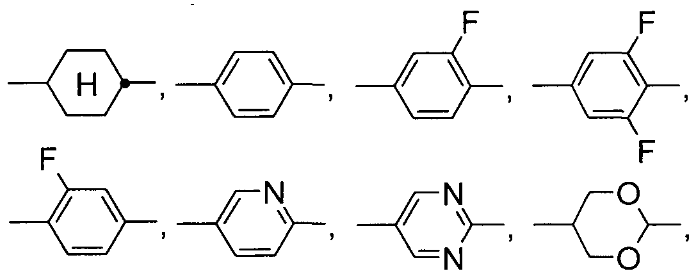

$Z^2$, $Z^3$, independently of one another, denote a single bond, $CF_2O$, $CH_2CH_2$, $CF_2CH_2$, $CF_2CF_2$, CFHCFH, $CFHCH_2$, (CO)O, $CH_2O$, C≡C, CH=CH, CF=CH, CF=CF, where asymmetrical bonding units may be oriented in both possible directions,

$X^1$ denotes F, Cl, CN, or alkyl, alkenyl, alkenyloxy, alkoxyalkyl or alkoxy having 1 to 3 C atoms which is mono- or polysubstituted by F, and

$L^1$ to $L^4$ denote H or F.

**8.** Use of the compounds of the formula I according to one or more of Claims 1 to 4 in a liquid-crystalline medium or in an electro-optical display.

**9.** Electro-optical display device containing a liquid-crystalline medium according to Claim 6 or 7.

**10.** Electro-optical display device according to Claim 9, **characterised in that** it operates entirely or partly in the region of the liquid-crystalline blue phase.

## Revendications

**1.** Composés de la formule I :

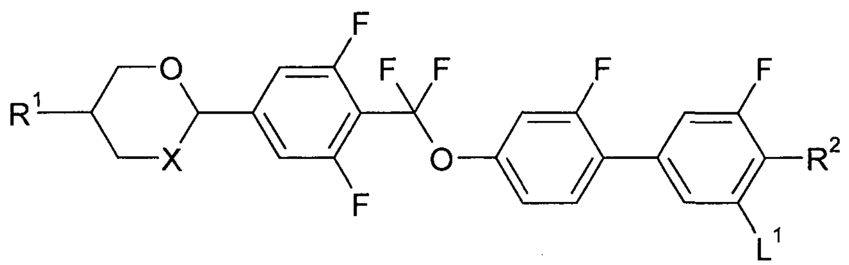

I

dans laquelle :

$L^1$ représente F,

X représente O,

$R^1$ représente un radical alkyle non substitué qui comporte 1 à 15 atome(s) de C, où, en outre, un ou plusieurs groupe(s) $CH_2$ dans ce radical peut/peuvent chacun être remplacé(s), indépendamment les uns des autres, par -C≡C-, -CH=CH-, -CF=CF-, -CF=CH-, -CH=CF-, -(CO)O-, -O(CO)-, -(CO)- ou -O- de telle sorte que des atomes de O ne soient pas liés directement les uns aux autres ,

et

$R^2$ représente $CF_3$ ou $OCF_3$.

**2.** Composés selon la revendication 1, **caractérisés en ce que** $R^2$ représente un groupe $CF_3$.

**3.** Composés selon une ou plusieurs des revendications 1 ou 2, **caractérisé en ce que** :

$R^1$ représente un radical alkyle qui comporte 1 à 9 atome(s) de C ou un radical alkényle qui comporte 2 à 9 atomes de C.

**4.** Composés selon une ou plusieurs des revendications 1 à 3, **caractérisés en ce que** :

$R^1$ représente un radical alkyle en chaîne droite qui comporte 3 atomes de C.

**5.** Procédé pour la préparation de composés de la formule I selon une ou plusieurs des revendications 1 à 4, incluant une étape de réaction selon laquelle deux matériaux de départ des formules A et B :

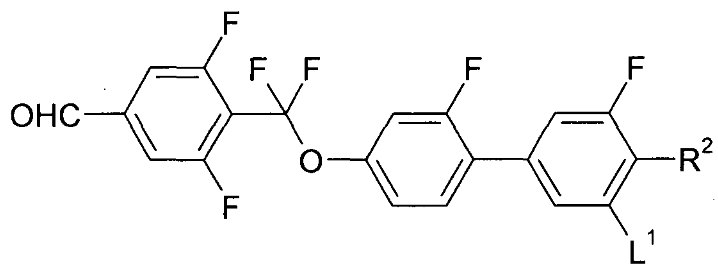

A

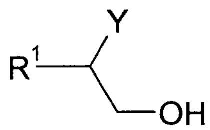

B

dans lesquelles $R^1$, $R^2$ et $L^1$ sont comme défini pour la formule I, et
Y représente un radical de la formule $-CH_2-OH$,

sont amenés à réagir sous des conditions de réaction appropriées.

**6.** Milieu cristallin liquide, **caractérisé en ce qu'**il comprend un ou plusieurs composé(s) de la formule I selon une ou plusieurs des revendications 1 à 4.

**7.** Milieu cristallin liquide selon la revendication 6, **caractérisé en ce qu'**il comprend de façon additionnelle un ou plusieurs composé(s) choisi(s) parmi les composés des formules II et III :

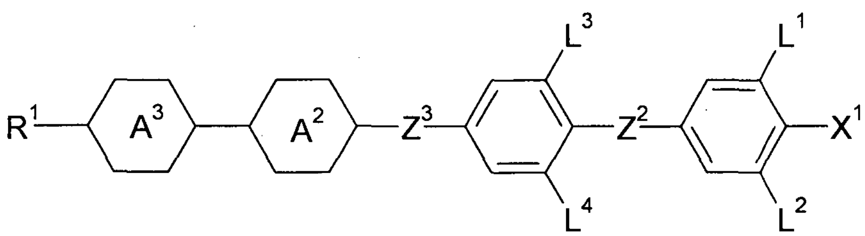

II

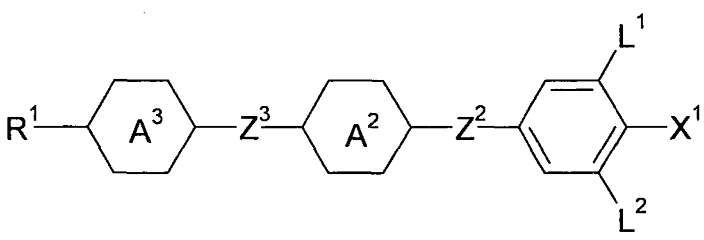

III

dans lesquelles :

$R^1$ représente, indépendamment les uns des autres, un radical alkyle non substitué qui comporte 1 à 15 atome(s) de C, où, en outre, un ou plusieurs groupe(s) $CH_2$ dans ce radical peut/peuvent chacun être remplacé(s), indépendamment les uns des autres, par -C≡C-, -CH=CH-, -CF=CF-, -CF=CH-, -CH=CF-, -(CO)O-, -O(CO)-, -(CO)- ou -O- de telle sorte que des atomes de O ne soient pas liés directement les uns aux autres,
$A^2$, $A^3$ représentent, indépendamment l'un de l'autre,

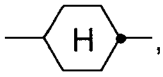, 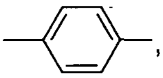, 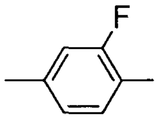, 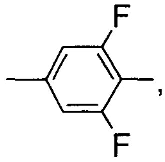,
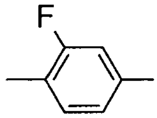, 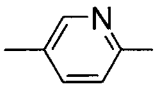, 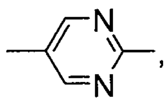, 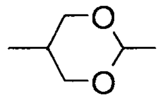,

$Z^2$, $Z^3$ représentent, indépendamment l'un de l'autre, une liaison simple, $CF_2O$, $CH_2CH_2$, $CF_2CH_2$, $CF_2CF_2$, CFHCFH, $CFHCH_2$, (CO)O, $CH_2O$, C≡C, CH=CH, CF=CH, CF=CF, où des unités de liaison asymétriques peuvent être orientées dans deux directions possibles,
$X^1$ représente F, Cl, CN, ou alkyle, alkényle, alkényloxy, alcoxyalkyle ou alcoxy comportant 1 à 3 atome(s) de C, lequel est monosubstitué ou polysubstitué par F, et
$L^1$ à $L^4$ représentent H ou F.

8. Utilisation des composés de la formule I selon une ou plusieurs des revendications 1 à 4 dans un milieu cristallin liquide ou dans un affichage électro-optique.

9. Dispositif d'affichage électro-optique contenant un milieu cristallin liquide selon la revendication 6 ou 7.

10. Dispositif d'affichage électro-optique selon la revendication 9, **caractérisé en ce qu'**il fonctionne en totalité ou partiellement dans la région de la phase bleue des cristaux liquides.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0819685 A1
- JP 10251186 A
- EP 2028252 A1
- WO 2004048501 A1
- US 20090237610 A1
- WO 2010134430 A1
- US 20090059157 A1
- WO 2006125526 A


### In der Beschreibung aufgeführte Nicht-Patentliteratur


- **H. KIKUCHI et al.** *Appl. Phys. Lett.,* 2008, vol. 92, 043119
- **J.O. METZGER et al.** *Bull. Soc. Chem. Belg.,* 1994, vol. 103, 393-7
- **B.C. JANU et al.** *Adv. Synth. Catal.,* 2004, vol. 346, 446-50
- **J.-Y. QI et al.** *Tetr. Lett.,* 2004, vol. 45, 7719-21
- **S.K. DE ; R.A. GIBBS.** *Tetr. Lett.,* 2004, vol. 45, 8141-4
- **WALLMICHRATH.** *Angew. Chem. Int. Ed.,* 2001, vol. 40, 1480-1484
- *Merck Liquid Crystals, Physical Properties of Liquid Crystals,* November 1997